# EUROPEAN PATENT APPLICATION

(11) **EP 1 074 545 A1**
(43) Date of publication of application: **07.02.2001**
(21) Application number: 98917644.1
(22) Date of filing: 23.04.1998
(51) Int. Cl.: C07D 217/02, C07D 217/24, C07D 401/12, A61K 31/495, A61K 31/55

(54) **ISOQUINOLINESULFONAMIDE DERIVATIVES AND DRUGS CONTAINING THE SAME AS THE ACTIVE INGREDIENT**

(71) Applicant: Hidaka, Hiroyoshi, Nagoya-shi, Aichi 468-0063 (JP)
(72) Inventor: HIDAKA, Hiroyoshi, Nagoya-shi Aichi 468-0063 (JP); MURAMATSU, Hiroshi, Hino-shi Tokyo 191-0055 (JP)
(74) Representative: HOFFMANN - EITLE
(86) International application number: JP9801868
(87) International publication number: WO9954306

(57) **Abstract**

This invention relates to isoquinolinesulfonamide derivatives represented by the following formula (1): 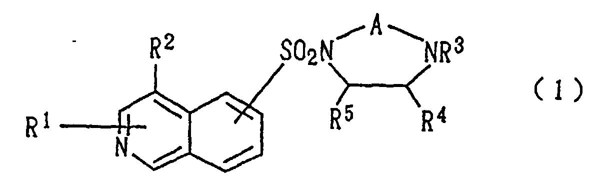 wherein A represents a linear or branched alkylene group, R¹ represents a hydrogen atom, an hydroxyl, alkoxy or alkyl group or the like, R² represents a hydrogen atom, an alkyl group or the like, R³ represents a hydrogen atom, an alkyl group or the like, and R⁴ and R⁵ may be the same or different and individually represent hydrogen atoms or lower alkyl groups; N-oxides and salts thereof; and solvates thereof. This invention is also concerned with pharmaceutical compositions, which contain the derivatives, N-oxides, salts, or solvates. These derivatives, N-oxides, salts, and solvates have viral proliferation inhibiting activities and are useful as AIDS therapeutics.

## Description

### Technical Field

This invention relates to isoquinolinesulfonamide derivatives excellent in antiviral activities, especially in anti-HIV activities and also to pharmaceuticals comprising the same as active ingredients.

### Background Art

As therapeutics for AIDS, nucleotidyl and non-nucleotidyl reverse transcriptase inhibitors, led by azidothymidine and zidovudine, and protease inhibitors, such as saquinavir and ritonavir, are used these days in addition to conventional virucides, and have achieved generally satisfactory results.

However, these pharmaceuticals are not considered to be weak in acute and chronic toxicity. Further, there are some reports to the effect that resistant virus strains have already appeared against these pharmaceuticals.

In addition, it is considered a difficulty for a reverse transcriptase inhibitor to immediately overcome AIDS because it depends on the biological characteristic of inducing an error when the corresponding reverse transcriptase replicates a viral genome.

Moreover, the conventional therapeutics are not of such a type as permitting restoration of immune function lost by HIV.

An object is therefore to provide a viral proliferation inhibitor, especially anti-HIV agents having a different acting mechanism and structure from the above-described existing pharmaceuticals.

### Disclosure of the Invention

With the foregoing circumstances in view, the present inventors were interested in p24 protein which makes up capsid as the shell of protein that protects the RNA of a virus, and postulated that the inhibition of the production of the protein would be able to prevent maturation of virus particles and hence to suppress proliferation of the virus in host cells. As a result of extensive research, the present inventors have found that the novel compounds represented by the below-described formula (1) suppress the production of p24 protein, prevent proliferation of viruses such as HIV, and are useful as virus proliferation inhibitors and AIDS therapeutics, leading to the completion of the present invention.

Namely, the present invention provides an isoquinolinesulfonamide derivative represented by the following formula (1): 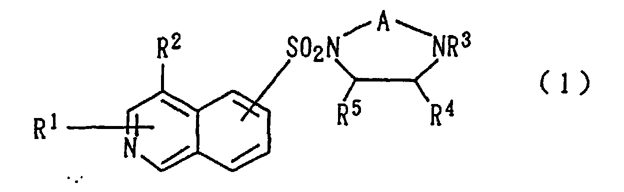 wherein A represents a linear or branched alkylene group, R¹ represents a hydrogen atom, a halogen atom, a hydroxyl group, an alkyl group, an alkoxy group, an aryloxy group, a benzyloxy group which may be substituted by one or more halogen atoms, an alkylthio group, an amino group, an alkylamino group, a dialkylamino group or -O(CH₂)ₙNH₂ in which n stands for an integer of from 2 to 6, R² represents a hydrogen atom, a halogen atom, an alkyl group or a cyano group, R³ represents a hydrogen atom, an alkyl group, an acetyl group or a benzyloxycarbonyl group, and R⁴ and R⁵ may be the same or different and individually represent hydrogen atoms or lower alkyl groups; an N-oxide or salt thereof; or a solvate thereof.

The present invention also provides a pharmaceutical which comprises as an active ingredient an isoquinolinesulfonamide derivative represented by the formula (1), an N-oxide or salt thereof, or a solvate thereof.

The present invention also provides an AIDS therapeutic which comprises as an active ingredient an isoquinolinesulfonamide derivative represented by the formula (1), an N-oxide or salt thereof, or a solvate thereof.

Moreover, the present invention also provides a viral proliferation inhibitor which comprises as an active ingredient an isoquinolinesulfonamide derivative represented by the formula (1), an N-oxide or salt thereof, or a solvate thereof.

The present invention also provides a pharmaceutical composition which comprises an isoquinolinesulfonamide derivative represented by the formula (1), an N-oxide or salt thereof, or a solvate thereof and a pharmacologically acceptable carrier.

The present invention also provides use of an isoquinolinesulfonamide derivative represented by the formula (1), an N-oxide or salt thereof, or a solvate thereof as a pharmaceutical.

The present invention also provides use of an isoquinolinesulfonamide derivative represented by the formula (1), an N-oxide or salt thereof, or a solvate thereof as an AIDS therapeutic.

The present invention also provides use of an isoquinolinesulfonamide derivative represented by the formula (1), an N-oxide or salt thereof, or a solvate thereof as a viral proliferation inhibitor.

The present invention also provides a treatment method for an HIV infectious disease, which comprises administering an effective amount of an isoquinolinesulfonamide derivative represented by the formula (1), an N-oxide or salt thereof, or a solvate thereof to a patient.

### Best Mode for Carrying out the Invention

As the linear or branched alkylene group represented by A in the formula (1), an alkylene group having 1-7 carbon atoms is preferred. Specific examples can include methylene, ethylene, trimethylene, tetramethylene, pentamethylene, hexamethylene, heptamethylene, 1-methylethylene, 1-methyltrimethylene, 2-methyltrimethylene, 1,1-dimethylethylene, 1,1-dimethyltrimethylene, 1,2-dimethyltrimethylene, and 2,2-dimethyltrimethylene groups. Of these, the alkylene groups having 1-5 carbon atoms are preferred, with the alkylene groups having 2-5 carbon atoms, for example, ethylene, trimethylene, 1-methylethylene, 1-methyltrimethylene, 2-methyltrimethylene, 2,2-dimethyltrimethylene groups and the like being particularly preferred. Preferred as the alkoxy group represented by R¹ is a linear or branched alkoxy group which has 1-20 carbon atoms and may contain one or more cycloalkyl groups in side chains. Specific examples can include methoxy, ethoxy, n-propoxy, i-propoxy, n-butoxy, i-butoxy, sec-butoxy, t-butoxy, hexyloxy, 2-ethylhexyloxy, octyloxy, octadecyloxy, and cyclohexylmethyloxy groups, among which i-propoxy, hexyloxy, 2-ethylhexyloxy and octadecyloxy groups can be exemplified as particularly preferred ones. As the benzyloxy group which may be substituted by one or more halogen atoms, a pentafluorophenylmethyloxy group or the like can be mentioned, for example. As the alkylthio group, an alkylthio group having 1-6 carbon atom can be mentioned. Specific examples can include methylthio and ethylthio groups. As the alkylamino group, an alkylamino group having 1-6 carbon atom can be mentioned. Specific examples can include methylamino, ethylamino and isopropylamino groups. Further, as the dialkylamino group, a di(C₁₋₆alkyl)amino group can be mentioned. Specific examples can include dimethylamino, diethylamino and di(n-propyl)amino groups. In addition, the alkyl groups represented by R¹, R² and R³ can preferably be alkyl groups having 1-6 carbon atoms. Specific examples can include methyl, ethyl, n-propyl, i-propyl, n-butyl, i-butyl, sec-butyl, tert-butyl, pentyl and hexyl groups. Illustrative of the halogen atoms can be fluorine, chlorine, bromine and iodine atoms. As the position of substitution by R¹, the 1-position or 3-position of the isoquinoline ring is referred, with the 1-position being particularly preferred. As the position of substitution by the sulfonamido group, on the other hand, the 5-position, 7-position or 8-position is preferred.

When the compounds represented by the formula (1) are used as pharmaceuticals, it is preferred to use the compounds which are obtained in Examples 6, 14, 20, 21, 22, 25, 28, 29, 36, 41, 46, 48, 51, 53, 55 ad 66 to be described subsequently herein.

No particular limitation is imposed on the salt of the compound according to the present invention, insofar as it is a pharmacologically acceptable salt. Illustrative of the salt can be inorganic acid salts such as the hydrochloride and the sulfate; and organic acid salts such as the fumarate, succinate, oxalate and acetate. As the solvate, the hydrate can be mentioned, for example. Some of the compounds according to the present invention may include isomers. Optically active substances and their mixtures shall also be included in the present invention.

The compound (1) can be prepared, for example, in accordance with the following reaction scheme. 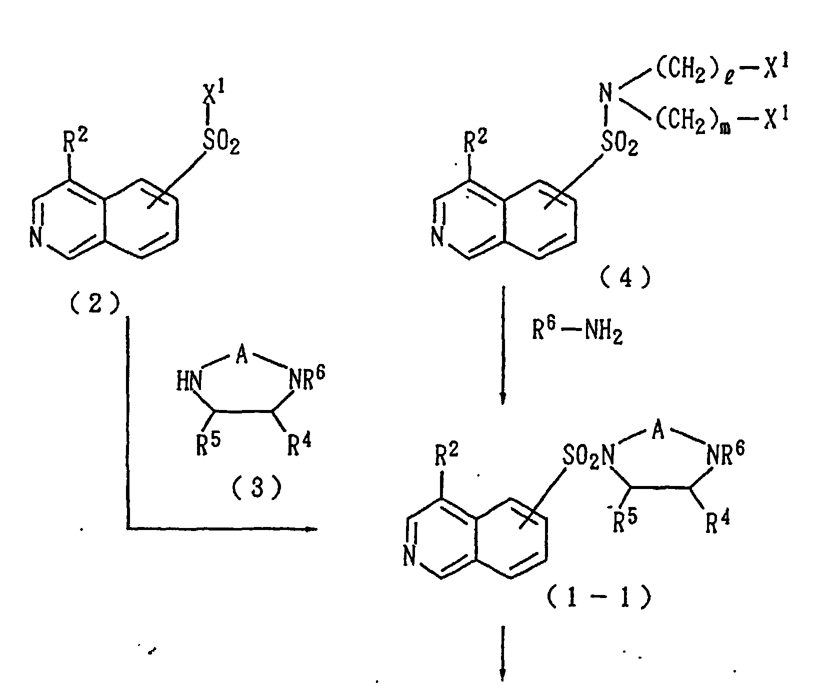 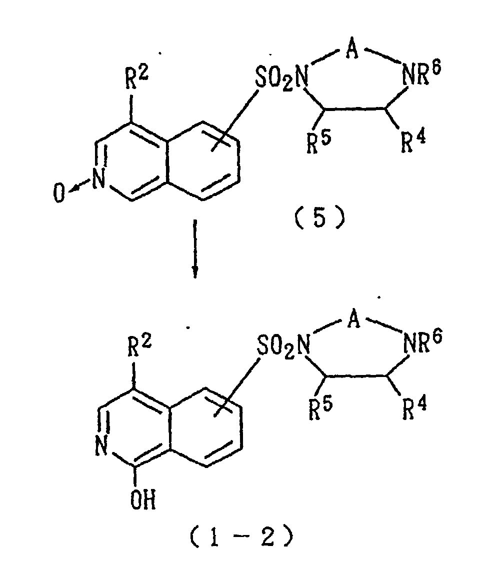 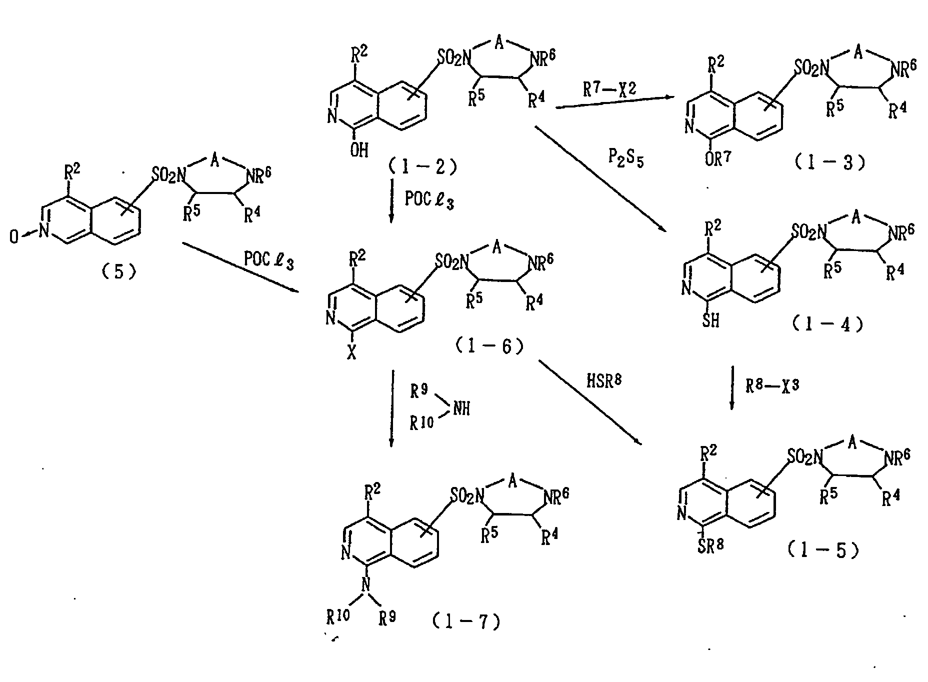 wherein R², R³, R⁴, R⁵ and A have the same meanings as defined above, R⁶ represents the same group as R³ or an amino-protecting group, R⁷ represents an alkyl group, an acyl group, an allyl group, a benzyl group which may be substituted by one or more halogen atoms, or -(CH₂)ₙNH₂ in which n stands for an integer of from 2 to 6, R⁸ represents an alkyl group, R⁹ and R¹⁰ may be the same or different and represent hydrogen atoms or alkyl groups, X¹, X² and X³ represents halogen atoms, and ℓ and m stand for integers of from 1 to 3, respectively.

Described specifically, a compound (1-1) is obtained either by reacting an isoquinoline derivative (2) with a cyclic amine compound (3) or by reacting an amine compound with a compound (4). The compound (1-1) is then oxidized into a compound (5), which is in turn converted into a compound (1-2) containing a hydroxyl group. Further, compounds (1-3) to (1-7) can be obtained by replacing the hydroxyl group of the compound (1-2) with other substituent groups by various methods.

The isoquinoline derivative (2), one of the raw materials, can be obtained by either chlorosulfonating or sulfonating and halogenating a 4-substituted isoquinoline which has been synthesized in accordance with a literature [Tetrahedron, **38**, 3347 (1982)] or as an alternative, by nitrating the 4-substituted isoquinoline with nitric acid and sulfuric acid, reducing the nitro group into an amino group and then conducting chlorosulfonation.

The reaction between the compound (2) and the compound (3) and the reaction between the compound (4) and the amine can be conducted preferably, for example, in the presence of a base such as sodium bicarbonate or sodium carbonate. The oxidation of the compound (1-1) can be conducted preferably by using hydrogen peroxide or the like. Further, the conversion of the compound (5) into the compound (1-2) can be conducted using acetic anhydride. The conversion from the compound (1-2) into the compound (1-3) can be conducted using an alkylating agent such as an alkyl iodide or diazomethane or an acylating agent such as an acyl halide. The conversion of the compound (1-2) into the compound (1-6) can be conducted using a halogenating agent such as phosphorus oxychloride or phosphorus pentachloride. The conversion of the compound (1-2) into the compound (1-4) can be conducted using phosphorus pentasulfide. The conversion of the compound (1-4) into the compound (1-5) can be conducted using an alkylating agent such as an alkyl iodide or diazomethane. In addition, the conversion of the compound (1-6) into the compound (1-7) can be conducted by reacting the compound (1-6) and an amine, if necessary, in the presence of a base.

The compound (1) obtained as described above has p24 protein production inhibiting activity and inhibits proliferation of viruses such as HIV, and can be used as pharmaceuticals such as an viral proliferation inhibitor and an AIDS therapeutic.

The compound (1) can be formulated together with pharmacologically acceptable carriers into pharmaceutical compositions of various preparation forms by methods known per se in the art. Its administration form is not specifically limited but can be selected as desired depending on the object of treatment. For example, its administration form can be any one of an oral preparation, an injection, a suppository and the like. These preparation forms can be obtained by formulation methods known to and commonly employed by those skilled in the present field of art.

To formulate a solid oral preparation, an excipient and, if necessary, a binder, a disintegrant, a lubricant, a coloring matter, a taste corrigent, a smell corrigent and/or the like can be added to the compound (1), and the resultant mixture can then be formed into tablets, coated tablets, granules, powder, capsules or the like by a method known *per se* in the art. Such additives can be those commonly used in the present field of art, including, for example, excipients such as lactose, sucrose, sodium chloride, glucose, starch, calcium carbonate, kaolin, microcrystalline cellulose and anhydrous silicic acid; binders such as water, ethanol, propanol, sucrose solution, glucose solution, starch solution, gelatin solution, carboxymethylcellulose, hydroxypropylcellulose, hydroxypropylstarch, methylcellulose, ethylcellulose, shellac, calcium phosphate and polyvinylpyrrolidone; disintegrators such as dry starch, sodium alginate, powdered agar, sodium bicarbonate, calcium carbonate, sodium laurylsulfate, stearic acid monoglyceride and lactose; lubricants such as purified talc, stearate salts, borax and polyethylene glycol; and taste corrigents such as sucrose, bitter orange peel, citric acid and tartaric acid.

To formulate a liquid oral preparation, a taste corrigent, a buffer, a stabilizer, a smell corrigent and the like can be added to the compound (1), and the resultant mixture can then be formed into a solution for internal use, a syrup, an elixir or the like. In this case, the taste corrigent can be those mentioned above, the buffer can be sodium citrate or the like, and the stabilizer can be tragacanth, gum arabic, gelatin or the like.

To formulate an injection, a pH regulator, a buffer, a stabilizer, an isotonicity, a local anesthetic and the like can be added to the compound (1), and the resultant mixture can then be formed into a subcutaneous, intramuscular or intravenous injection by a method known *per se* in the art. Examples of the pH regulator and buffer can include sodium citrate, sodium acetate, and sodium phosphate. Examples of the stabilizer can include sodium pyrosulfite, EDTA, thioglycolic acid, and thiolactic acid. Illustrative of the local anesthetic can be procaine hydrochloride and lidocaine hydrochloride. Illustrative of the isotonicity can be sodium chloride and glucose.

To formulate suppositories, a pharmaceutical carrier known in the present field of art, for example, polyethylene glycol, lanolin, cacao butter, a fatty acid triglyceride or the like and, if necessary, a surfactant such as "Tween" (trade mark) can be added to the compound (1), and the resulting mixture can then be formed into suppositories by a method known *per se* in the art.

The amount of the compound (1) to be incorporated in each of the above administration unit forms varies depending on the conditions of a patient to whom the administration unit form is applied, the preparation form and the like but, per administration unit form, may generally be desired to range from about 1 to 500 mg in the case of oral preparations, from about 1 to 200 mg in the case of injections, and from about 1 to 500 mg in the case of suppositories. Further, the daily dose of the pharmaceutical in each of the abovedescribed administration forms varies depending on the conditions, weight, age, sex and the like of the patient and cannot be determined in any wholesale manner. It is however generally necessary to set the daily dose at about 0.1 to 25 mg/kg-adult, preferably about 0.1 to 20 mg/kg-adult. It is preferred to administer this dose either at once or in 2 to 4 portions or so in a day.

### Examples

The present invention will next be described in further detail by Examples. It should however be borne in mind that the present invention is by no means limited to them.

### Referential Example 1

### 4-Methyl-5-nitroisoquinoline

4-Methyl-5-isoquinoline (19 g) was added to concentrated sulfuric acid (68 mℓ), followed by the dropwise addition of a solution of potassium nitrate (13.5 g) in concentrated sulfuric acid (51 mℓ) under stirring at 0°C. After the resulting mixture was stirred for 30 minutes under the same conditions, the mixture was poured into ice water, neutralized with aqueous ammonia, and then extracted with ethyl acetate. The extract was washed with a saturated aqueous solution of sodium chloride and then dried over anhydrous magnesium sulfate. The solvent was distilled off under reduced pressure. The resulting residue was subjected to chromatography on a silica gel column. From fractions eluted with hexane-ethyl acetate (1:1), 18.5 g of the target compound were obtained as pale yellow crystals.
¹H-NMR (270 MHz, CDCℓ₃) δ:
   9.23(1H,s), 8.50(1H,s), 8.18(1H,dd,J=8.25,0.99Hz), 7.89(1H,d,J=7.59Hz), 7.66(1H,t,J=7.59Hz), 2.50(3H,s).

### Referential Example 2 (5-Amino-methylisoquinoline)

### 5-Amino-5-methylisoquinoline

4-Methyl-5-nitroisoquinoline (7.1 g) was dissolved in methanol (100 mℓ). Under stirring at room temperature, tin (5.4 g) and concentrated hydrochloric acid (2 mℓ) were added, followed by stirring for 5 hours at the same temperature. After the solvent was distilled off under reduced pressure and the concentrate was neutralized with an aqueous solution of sodium bicarbonate, the concentrate was extracted with ethyl acetate. The extract was washed with a saturated aqueous solution of sodium chloride and was then dried over anhydrous magnesium sulfate. The solvent was distilled off under reduced pressure. The resulting residue was subjected to chromatography on a silica gel column. From fractions eluted with chloroform-acetone (9:1), 5.5 g of the target compound were obtained as pale yellow crystals.
¹H-NMR (270 MHz, CDCℓ₃) δ:
   8.96(1H,s), 8.14(1H,s), 7.34-7.36(2H,m), 6.85-6.88(1H,m), 4.41(2H,brs), 2.91(3H,s).

### Referential Example 3

### 4-Methyl-5-isoquinolinesulfonyl chloride

5-Amino-4-methylisoquinoline (2.24 g) was added to concentrated hydrochloric acid (10 mℓ), followed by the dropwise addition of a solution of sodium nitrite (1.64 g) in water (10 mℓ) under stirring over an ice bath. The resultant mixture was stirred for 1 hour. The reaction mixture was added dropwise to a mixed solution consisting of acetic acid (10 mℓ), into which sulfurous acid had been introduced, and cupric chloride (2.76 g). After the resultant mixture was stirred for 1 hour, the solvent was distilled off under reduced pressure and, subsequent to neutralization with an aqueous solution of sodium bicarbonate, the concentrate was extracted with chloroform. The extract was washed with a saturated aqueous solution of sodium chloride and dried over magnesium sulfate, and the solvent was then distilled off under reduced pressure. Crude crystals so obtained were recrystallized form benzene, whereby 1.7 g of the target compound were obtained as pale yellow crystals.
¹H-NMR (270 MHz, CDCℓ₃) δ:
   9.23(1H,s), 8.86(1H,s), 8.85(1H,dd,J=7.59,1.32Hz), 8.36(1H,dd,J=7.92,1.32Hz), 7.75(1H,t,J=7.92Hz), 3.16(3H,s).

### Referential Example 4

### 4-Fluoro-5-isoquinolinesulfonyl chloride

From 4-bromoisoquinoline (75.0 g), 4-aminoisoquinoline (39.8 g) was synthetically obtained following J. Am. Chem. Soc., **64**, 783 (1942). From 4-aminoisoquinoline (20.0 g), 4-fluoroisoquinoline (9.61 g) was obtained following J. Am. Chem. Soc., **73**, 683 (1951). 4-Fluoroisoquinoline was treated in a similar manner as in Referential Examples 1, 2 and 3.
¹H-NMR (270 MHz, CDCℓ₃) δ:
   9.25(1H,s), 8.73(1H,d,J=4.45Hz), 8.73(1H,dd,J=7.59,0.99Hz), 8.42(1H,ddd,J=8.25,1.82,1.16Hz), 7.84(1H,t,J=7.91Hz).

### Referential Example 5

### 5-Methylhexahydro-1H-1,4-diazepine

1,3-Dibromobutane (7.5 g) was added to a solution of N,N'-dibenzylethylenediamine (8.4 g) and triethylamine (7.0 g) in 200 mℓ of benzene. The resultant mixture was heated for 48 hours under reflux. After cooling, the reaction mixture was washed with water and a saturated solution of sodium chloride and was then dried over anhydrous sodium sulfate. The solvent was distilled off under reduced pressure, and the resultant residue was subjected to chromatography on a silica gel column. From fractions eluted with ethyl acetate, a pale brown oil was obtained (5.0 g). The oil was dissolved in 50 mℓ of ethanol. Subsequent to addition of 0.8 g of 5% palladium carbon, the solution so obtained was stirred at room temperature in a hydrogen gas atmosphere. After completion of the reaction, the solvent was distilled off under reduced pressure, whereby 2.0 g of the target compound were obtained as a pale yellow oil.
¹H-NMR (270 MHz, CDCℓ₃) δ:
   2.73-3.09(7H,m), 2.02(2H,s), 1.81-1.92(1H,m), 1.35-1.49(1H,m), 1.13(3H,d,J=6.26Hz).

### Referential Example 6

### 1-Benzyloxycarbonyl-5-methylhexahydro-1H-1,4-diazepine

To a solution of 5-methylhexahydro-1H-1,4-diazepine (1.7 g) and triethylamine (1.5 g) in 20 mℓ of dichloromethane, a solution of carbobenzoxy chloride (2.0 g) in 2 mℓ of dichloromethane was slowly added dropwise under ice cooling and stirring. After completion of the reaction, the reaction mixture was washed with water and a saturated aqueous solution of sodium chloride and was then dried over anhydrous sodium sulfate. After the solvent was distilled off under reduced pressure, the resultant residue was subjected to chromatography on an NH-silica gel column. From fractions eluted with ethyl acetate, 2.6 g of the target compound were obtained as a pale yellow oil.
¹H-NMR (270 MHz, CDCℓ₃) δ:
   7.29-7.36(5H,m), 5.13(2H,s), 3.53-3.76(2H,m), 3.29-3.48(2H,m), 3.03-3.14(1H,m), 2.67-2.86(2H,s), 1.79-1.92(1H,m), 1.37-1.58(2H,m), 1.10(3H,dd,J=6.26,1.98Hz).

### Referential Example 7

### 4-Methyl-8-isoquinolinesulfonic acid·1/2 sulfate

4-Methylisoquinoline (9.96 g) was carefully added to 25% fuming sulfuric acid (10 mℓ) with stirring under ice cooling. The reaction mixture was gradually heated to 80°C and was then stirred for 2 hours at the same temperature. After cooling, the reaction mixture was carefully poured into about 200 g of ice water, and precipitated crystals were collected by filtration. The crystals so collected by filtration were washed with chilled water and then dried under reduced pressure, whereby 9.44 g of the target compound were obtained.
¹H-NMR (270 MHz, d₆-DMSO) δ:
   10.24(1H,s), 8.64(1H,s), 8.35(1H,t,J=8.58Hz), 8.20-8.31(2H,m), 2.80(3H,s).

### Referential Example 8

### Ethyl 2-(3-aminopropylamino)propionate

Chloroform (500 mℓ) was added to 1,3-diaminopropane (110.1 g) for dissolution. Ethyl 2-bromopropionate (89.59 g) was added to the thus-obtained solution, followed by stirring at 65°C for 3 hours. The reaction mixture was allowed to cool down and was then transferred into a separation funnel. The reaction mixture was washed with water, and the water layer was extracted with chloroform (50 mℓ x 2). Chloroform layers were combined, washed with a saturated aqueous solution of sodium chloride, and then dried over anhydrous magnesium sulfate. Subsequent to filtration, the solvent was distilled off under reduced pressure. The thus-obtained residue was provided for use in the next reaction without purification.
¹H-NMR (270 MHz, CDCℓ₃) δ:
   4.18(2H,dq,J=6.7,3.0Hz), 3.28-3.38(2H,m), 2.50-2.75(3H,m), 1.67(2H,m), 1.29(3H,d,J=6.6Hz), 1.28(3H,dt,J=6.7,1.6Hz).

### Referential Example 9

### 3-Methyl-hexahydro-1H-1,4-diazepin-2-one

To the crude product (86.2 g) obtained in Referential Example 8, 670 mℓ of toluene and 31 g of triethylamine were added, and the resultant mixture was heated for 15 hours under reflux. After the reaction mixture was allowed to cool down, the solvent and the triethylamine were distilled off under reduced pressure. The residue so obtained was subjected to chromatography on a silica gel column (chloroform: methanol = 20:1). Fractions of mixtures containing the target compound were concentrated and, subsequent to addition of 30 mℓ of ethyl acetate to the thus-obtained concentrate, the resulting mixture was allowed to stand. Precipitated crystals were collected by suction filtration and then washed with 50 mℓ of n-hexane:ethyl acetate (3:1), whereby 7.43 g of the target compound were obtained as colorless crystals.
¹H-NMR (270 MHz, CDCℓ₃) δ:
   5.96(1H,br), 3.43(1H,q,J=6.7Hz), 3.22-3.40(3H,m), 2.95(1H,ddd,J=13.5,11.1,3.3Hz), 1.58-1.79(2H,m), 1.29(3M,d,J=6.6Hz).

### Referential Example 10

### 2-Methyl-hexahydro-1H-1,4-diazepine

Tetrahydrofuran (200 mℓ) was added to 10.00 g of the produce obtained in Referential Example 9, followed by the addition of 7.00 g of lithium aluminum hydride in portions at room temperature. After an exothermic reaction subsided, the reaction mixture was stirred at 50°C to 60°C for 2 hours. The reaction mixture was allowed to cool down and was then cooled over an ice water bath. A 20% aqueous solution of sodium hydroxide (10 mℓ) and a 2 N aqueous solution of sodium hydroxide (10 mℓ) were alternately added slowly with care. A white precipitate so formed was removed by suction filtration and then washed with tetrahydrofuran. The filtrate and the washing were combined and then concentrated. As a result, the target compound containing the raw materials at low concentrations was obtained as a colorless oil in an approximately stoichiometric amount. This compound was provided for use in the next reaction without purification.
¹H-NMR (270 MHz, CDCℓ₃) δ:
   2.76-3.11(6H,m), 2.43(1H,dd,J=13.5,8.9Hz), 1.67-1.83(2H,m), 1.03(3H,d,J=6.6Hz).

### Referential Example 11

### 1-t-Butoxycarbonyl-3-methyl-hexahydro-1H-1,4-diazepine

Chloroform (200 mℓ) was added to the whole crude product obtained in Referential Example 10, to which a solution of di(t-butyl) bicarbonate (17.46 g) in 50 mℓ of chloroform was added dropwise over 10 minutes under ice water cooling. Subsequent to stirring for 30 minutes, the solvent was distilled off under reduced pressure. The residue so obtained was subjected to chromatography on a silica gel column (chloroform: methanol = 100:3), whereby 13.19 g of the target compound were obtained as colorless crystals.
¹H-NMR (270 MHz, CDCℓ₃) δ:
   3.65-3.91(2H,m), 3.03-3.28(2H,m), 2.85-3.00(1H,m), 2.45-2.77(2H,m), 1.64-2.00(2H,m), 1.46(9H,s), 1.06(3H,d,J=6.6Hz).

### Referential Example 12

### 1-t-Butoxycarbonyl-3-ethyl-hexahydro-1H-1,4-diazepine

Using 25.90 g of methyl 2-bromobutyrate in lieu of ethyl 2-bromopropionate, a reaction was conducted with 40.0 g of 1,3-diaminopropane. The reaction mixture was then treated as in Referential Examples 9, 10 and 11, whereby 5.03 g of the target compound were obtained.
¹H-NMR (270 MHz, CDCℓ₃) δ:
   3.63-3.95(2H,m), 3.05-3.25(2H,m), 2.52-2.85(3H,m), 1.65-2.03(2H,m), 1.47(9H,s), 1.32-1.47(2H,m), 0.96(3H,t,J=7.6Hz).

### Referential Example 13

### 1-t-Butoxycarbonyl-3-methyl-6,6-dimethyl-hexahydro-1H-1,4-diazepine

Using 25.00 g of 2,2-dimethyl-1,3-diaminopropane in place of 1,3-diaminopropane, a reaction was conducted with 22.2 g of 2-bromopropionate. The reaction mixture was then treated in a similar manner, whereby 4.91 g of the target compound were obtained.
¹H-NMR (270 MHz, CDCℓ₃) δ:
   3.91(2/3H,dd,J=13.5,3.6Hz), 3.62(1/3H,dd,J=13.5,3.6Hz), 3.45(2/3H,d,J=14.2Hz), 3.45(1/3H,d,J=14.2Hz), 3.04(1/3H,d,J=14.2Hz), 2.88(2/3H,d,J=14.2Hz), 2.75-2.95(1H,m), 2.64(2/3H,d,J=13.5Hz), 2.58(1/3H,d,J=13.2Hz), 2.54(1/3H,d,J=13.2Hz), 2.45(2/3H,d,J=13.5Hz), 1.46(9H,s), 1.04(3H,d,J=6.6Hz),0.90(6H,d,J=6.0Hz).

### Referential Example 14

### 1,4-Dimethylisoquinoline-7-sulfonyl chloride

Chlorosulfonic acid (2.0 mℓ) was added to 1,4-dimethylisoquinoline (622.6 mg) under ice cooling, followed by stirring at 80°C for 12 hours. The reaction mixture was poured into 50 g of ice water. The resultant mixture was alkalized with sodium bicarbonate and then extracted with ethyl acetate (50 mℓ x 3). The extracts were dried over anhydrous magnesium sulfate and the solvent was distilled off under reduced pressure, whereby 300.0 mg of the target compound was obtained.
¹H-NMR (270 MHz, CDCℓ₃) δ:
   9.04(1H,d,J=1.65Hz), 8.62(1H,dd,7=9.07,1.64Hz), 8.52(1H,s), 8.47(1H,d,J=9.24Hz), 3.51(3H,s), 2.85(3H,s).

### Example 1

### 1-(4-Methyl-5-isoquinolinesulfonyl)hexahydro-1H-1,4-diazepine·dihydrochloride

To 100 mℓ of chloroform-water (2:1), 1-t-butoxycarbonylhexahydro-1H-1,4-diazepine (2.0 g) and sodium bicarbonate (1.0 g) were added. Under stirring over an ice bath, the 4-methyl-5-isoquinolinesulfonyl chloride (2.41 g) obtained in Referential Example 3 was added, followed by stirring for 3 hours. The resultant mixture was extracted with chloroform. After the extract was washed with a saturated aqueous solution of sodium chloride and dried over magnesium sulfate, the solvent was distilled off under reduced pressure. The thus-obtained residue subjected to chromatography on a silica gel column, followed by elution with hexane-ethyl acetate (5:1). Pale yellow crystals (3.67 g) were obtained. These crystals were dissolved in ethanol (850 mℓ), to which 1 N hydrochloric acid (40 mℓ) was added. The resulting mixture was stirred for 1 hour under reflux and was then concentrated. Water was added to the concentrate, and the resulting mixture was alkalized with sodium bicarbonate and then extracted with chloroform. The extract was dried over magnesium sulfate and the solvent was distilled off under reduced pressure, whereby white crystals were obtained (1.85 g). The crystals were dissolved in chloroform, followed by the addition of ethanol which had been saturated with hydrogen chloride gas. The resultant mixture was concentrated, and precipitated crystals were then collected by filtration, whereby 1.93 g of the target compound were obtained.
¹H-NMR (270 MHz, d₆-DMSO) δ:
   9.74(1H,s), 8.70(1H,s), 8.68(1H,d,J=10.89Hz), 8.36(1H,d,J=7.26Hz), 7.98(1H,t,J=7.91Hz), 3.88(2H,brs), 3.67(2H,brs), 3.53(2H,brs), 3.29(2H,brs), 3.05(3H,s), 2.21(2H,brs).

### Example 2

### 1-(4-Methyl-5-isoquinolinesulfonyl)-2-ethylhexahydro-1H-1,4-diazepine

Using as an amine the 1-t-butoxycarbonyl-3-ethylhexahydro-1H-1,4-diazepine synthesized in Referential Example 12, treatments were conducted as in Example 1, whereby 342.0 mg of the target compound were obtained.
¹H-NMR (270 MHz, CDCℓ₃) δ:
   9.12(1H,s), 8.85(1H,d,J=7.59Hz), 8.53(1H,s), 8.13(1H,d,J=7.92Hz), 7.59(1H,t,J=7.76Hz), 3.91(2H,m), 3.40(2H,m), 3.23(1H,m), 3.10(3H,s), 2.75(2H,m), 2.07(1H,m), 1.82(3H,m), 0.83(3H,t,J=7.59Hz).

### Example 3

### 1-(4-Methyl-5-isoquinolinesulfonyl)-2-methyl-6,6-dimethyl-hexahydro-1H-1,4-diazepine

Using as an amine the 1-t-butoxycarbonyl-3-methyl-6,6-dimethylhexahydro-1H-1,4-diazepine synthesized in Referential Example 13, treatments were conducted as in Example 1, whereby 69.0 mg of the target compound were obtained.
¹H-NMR (270 MHz, CDCℓ₃) δ:
   9.13(1H,s), 8.54(1H,s), 8.28(1H,m), 8.13(1H,m), 7.58(1H,t,J=7.76Hz), 4.16(1H,m), 3.47(1H,d,J=14.85Hz), 3.26(2H,m), 3.10(3H,s), 2.71(2H,m), 2.56(1H,d,J=13.20Hz), 1.22(3H,d,J=6.60Hz), 0.99(3H,s), 0.93(3H,s).

### Example 4

### 1-(4-Methyl-5-isoquinolinesulfonyl)-2-methylhexahydro-1H-1,4-diazepine

Using as an amine the 1-t-butoxycarbonyl-3-methyl-hexahydro-1H-1,4-diazepine synthesized in Referential Example 11, treatments were conducted as in Example 1, whereby 200.0 mg of the target compound were obtained.
¹H-NMR (270 MHz, CDCℓ₃) δ:
   9.19(1H,s), 8.86(1H,d,J=7.26Hz), 8.60(1H,s), 8.18(1H,d,J=7.92Hz), 7.65(1H,t,J=7.92Hz), 4.13-4.31(1H,m), 3.87-4.03(1H,m), 3.34-3.49(2H,m), 3.20-3.34(1H,m), 3.17(3H,s), 2.77-2.89(1H,m), 2.68(1H,dd,J=9.24,14.52Hz), 1.97-2.20(1H,m), 1.66-1.81(1H,m), 1.27(3H,d,J=6.27Hz).
Mass (FAB) m/z for C₁₆H₂₁N₃O₂S: 320 (M+H⁺)

### Example 5

### 1-(1-Hydroxy-4-methyl-5-isoquinolinesulfonyl)hexahydro-1H-1,4-diazepine·monohydrochloride

1-(4-Methyl-5-isoquinolinesulfonyl)hexahydro-1H-1,4-diazepine (3 g) was added to a mixture consisting of pyridine (30 mℓ) and acetic anhydride (15 mℓ), followed by stirring under heating at 60°C for 30 minutes. Water (30 mℓ) was then added. The resulting mixture was stirred for 30 minutes and was then extracted with chloroform. The extract was washed with a saturated aqueous solution of sodium chloride and then dried over magnesium sulfate. The solvent was distilled off under reduced pressure, whereby 4-acetyl-1-(4-methyl-5-isoquinolinesulfonyl)hexahydro-1H-1,4-diazepine (3.1 g) was obtained. This compound (2.87 g) was dissolved in acetic acid (40 mℓ), followed by the addition of a 30% aqueous solution of hydrogen peroxide (1.4 g). The mixture so obtained was stirred overnight under heating at 60°C. The reaction mixture was poured into ice water, the resulting mixture was alkalized with potassium carbonate, and precipitated crystals were collected by filtration, whereby a 2-oxy compound (2.9 g) was obtained. This compound (2.8 g) was added to acetic anhydride (50 mℓ), followed by heating under reflux for 5 hours. The reaction mixture was concentrated under reduced pressure and the residue was dissolved in methanol (30 mℓ). A 10% aqueous solution of sodium hydroxide (15 mℓ) was added further. The mixture so obtained was stirred at room temperature for 1 hour and then poured into water. The thus-obtained mixture was acidified with citric acid and then extracted with chloroform. The extract was washed with a saturated aqueous solution of sodium chloride and then dried over magnesium sulfate. The solvent was distilled under reduced pressure. The resulting residue was purified by chromatography on a silica gel column (chloroform:methanol = 20:1), whereby 1-acetyl-4-(1-hydroxy-4-methyl-5-isoquinolinesulfonyl)hexahydro-1H-1,4-diazepine (1.95 g) was obtained. This compound was added to 1 N hydrochloric acid (30 mℓ). The resultant mixture was stirred overnight under heating, alkalized with sodium bicarbonate, and then extracted with chloroform. The extract was dried over magnesium sulfate, and the solvent was distilled off under reduced pressure. The thus-obtained residue was purified by chromatography on a silica gel column (chloroform: methanol = 4:1), whereby 1-(1-hydroxy-4-methyl-5-isoquinolinesulfonyl)hexahydro-1H-1,4-diazepine (0.83 g) was obtained. It was treated as in Example 1, whereby 0.85 g of the target compound was obtained.
¹H-NMR (270 MHz, d₆-DMSO) δ:
   11.75(1H,brs), 8.66(1H,d,J=7.92Hz), 8.02(1H,d,J=7.92Hz), 7.70(1H,t,J=7.90Hz), 7.28(1H,m), 3.80-4.00(2H,m), 3.60-3.80(2H,m), 3.30-3.50(4H,m), 2.60(3H,m), 2.10-2.30(2H,m).

### Example 6

### 1-(1-Hydroxy-5-isoquinolinesulfonyl)hexahydro-1H-1,4-diazepine·monohydrochloride

Using 5-isoquinolinesulfonyl chloride, treatments were conducted as in Examples 1 and 5, whereby 100.3 mg of the target compound were obtained.
¹H-NMR (270 MHz, d₆-DMSO) δ:
   11.64(1H,brd), 9.11(1H,brs), 8.43(1H,dd,J=7.92,1.32Hz), 8.13(1H,dd,J=7.92,1.32Hz), 7.56(1H,t,J=7.91Hz), 7.33(1H,dd,J=7.92,5.94Hz), 3.50-3.60(2H,m), 3.35-3.45(2H,m), 3.05-3.15(4H,m), 1.90-2.00(2H,m).

### Example 7

### 1-(4-Methyl-5-isoquinolinesulfonyl)hexahydro-1H-1,4-diazepine

To a solution of hexahydro-1H-1,4-diazepine (4.88 g) in chloroform (100 mℓ), a solution of 4-methyl-5-isoquinolinesulfonyl chloride (4.00 g) in chloroform (30 mℓ) was added dropwise over 15 minutes with stirring under ice cooling. After completion of the dropwise addition, the resulting mixture was stirred at room temperature for 14 hours. The reaction mixture was washed first with water (100 mℓ x 2) and then with a saturated aqueous solution of sodium chloride (100 mℓ), and was then dried over anhydrous magnesium sulfate. The solvent was distilled off under reduced pressure. The residue was purified by chromatography on a silica gel column (chloroform: methanol = 100:1), whereby 4.32 g of the target compound were obtained.
¹H-NMR (270 MHz, CDCℓ₃) δ:
   9.14(1H,s), 8.55(1H,s), 8.15(1H,dd,J=8.08,1.32Hz), 7.85(1H,dd,J=7.67,1.31Hz), 7.60(1H,t,J=7.92Hz), 3.66(2H,t,J=5.94Hz), 3.59(2H,t,J=5.27Hz), 3.09(3H,s), 3.08-3.12(4H,m), 1.93-2.01(2H,m).

### Example 8

### 4-Benzyloxycarbonyl-1-(4-methyl-5-isoquinolinesulfonyl)hexahydro-1H-1,4-diazepine

To a solution of 1-(4-methyl-5-isoquinolinesulfonyl)hexahydro-1H-1,4-diazepine (1.38 g) in dimethylformamide (15 mℓ), potassium carbonate (0.83 g) and benzyl chloroformate (0.80 mℓ) were successively added under ice cooling. The resulting mixture was stirred at room temperature for 2.5 hours. Ethyl acetate (200 mℓ) was added to the reaction mixture. The mixture so obtained was washed first with water (100 mℓ x 2) and then with a saturated aqueous solution of sodium chloride (100 mℓ), and was then dried over anhydrous magnesium sulfate. The solvent was distilled off under reduced pressure. The residue was then purified by chromatography on a silica gel column (chloroform:methanol = 100:1), whereby 1.98 g of the target compound were obtained.
¹H-NMR (270 MHz, CDCℓ₃) δ:
   9.18(1H,s), 8.55(1H,s), 8.19(1H,d,J=7.92Hz), 7.77(1H,d,J=7.59Hz), 7.62(1H,t,J=7.26Hz), 7.38(5H,m), 5.20(2H,s), 3.72(4H,brs), 3.49-3.58(4H,m), 3.09(3H,s), 2.05-2.14(2H,m).

### Example 9

### 4-Benzyloxycarbonyl-1-(4-methyl-2-oxy-5-isoquinolinesulfonyl)hexahydro-1H-1,4-diazepine

To a solution of 4-benzyloxycarbonyl-1-(4-methyl-5-isoquinolinesulfonyl)hexahydro-1H-1,4-diazepine (5.46 g) in acetic acid (30 mℓ), a 31% aqueous solution of hydrogen peroxide (1.40 g) was added, followed by stirring at 70°C for 12 hours. The reaction mixture was poured into water (400 mℓ). The thus-obtained mixture was alkalized with potassium carbonate and then extracted with chloroform (300 mℓ x 3). The extracts were washed with a saturated aqueous solution of sodium chloride (200 mℓ) and then dried over anhydrous magnesium sulfate. The solvent was then distilled off under reduced pressure. The resulting residue was purified by chromatography on a silica gel column (chloroform:methanol = 100:1), whereby 5.37 g of the target compound were obtained.
¹H-NMR (270 MHz, CDCℓ₃) δ:
   8.69(1H,d,J=1.32Hz), 8.17(1H,s), 7.86(1H,dd,J=7.43,1.98Hz), 7.54-7.65(2H,m), 7.38(5H,m), 5.19(2H,s), 3.71(4H,brs), 3.47-3.62(4H,m), 3.04(3H,s), 2.03-2.14(2H,m).

### Example 10

### 4-Benzyloxycarbonyl-1-(1-hydroxy-4-methyl-5-isoquinolinesulfonyl)hexahydro-1H-1,4-diazepine

A solution of 4-benzyloxycarbonyl-1-(4-methyl-2-oxy-5-isoquinolinesulfonyl)hexahydro-1H-1,4-diazepine (5.37 g) in acetic anhydride (20 mℓ) was stirred at 140°C for 11 hours. The solvent was distilled off under reduced pressure, and methanol (100 mℓ) was added to the residue for dissolution. The thus-obtained solution was added to a 1 N aqueous solution of sodium hydroxide (15.0 mℓ), followed by stirring at 60°C for 30 minutes. The solvent was distilled off under reduced pressure. A 1 N aqueous solution of hydrochloric acid was added to the residue to acidify the latter. The thus-acidified residue was then extracted with ethyl acetate (200 mℓ x 3). The extracts were washed with a saturated aqueous solution of sodium chloride (200 mℓ) and then dried over anhydrous magnesium sulfate. The solvent was then distilled off under reduced pressure. The resulting residue was purified by chromatography on a silica gel column (chloroform:methanol = 50:1), whereby 3.83 g of the target compound were obtained.
¹H-NMR (270 MHz, CDCℓ₃) δ:
   8.76(1H,d,J=7.91Hz), 7.67(1H,d,J=7.58Hz), 7.50(1H,t,J=7.92Hz), 7.38(5H,m), 7.10(1H,s), 5.19(2H,s), 3.70(4H,brs), 3.47-3.58(4H,m), 2.72(3H,s), 1.99-2.12(2H,m).

### Example 11

### 4-Benzyloxycarbonyl-1-(1-methoxy-4-methyl-5-isoquinolinesulfonyl)hexahydro-1H-1,4-diazepine

To a solution of 4-benzyloxycarbonyl-1-(1-hydroxy-4-methyl-5-isoquinolinesulfonyl)hexahydro-1H-1,4-diazepine (140.0 mg) in dimethylformamide (2.0 mℓ), 60% sodium hydride (20.0 mg) was added under ice cooling. At the same temperature, the thus-obtained mixture was stirred for 20 minutes. Iodomethane (0.02 mℓ) was added to the reaction mixture under ice cooling, followed by stirring at room temperature for 30 minutes. The reaction mixture was poured into 0.1 g of ice water with care, and the resulting mixture was acidified with a 1 N aqueous solution of hydrochloric acid and then extracted with ethyl acetate (100 mℓ x 3). The extracts were washed with a saturated aqueous solution of sodium chloride (100 mℓ) and then dried over anhydrous magnesium sulfate. The solvent was then distilled off under reduced pressure. The resulting residue was purified by chromatography on a silica gel column (chloroform:methanol = 100:1), whereby 90.0 mg of the target compound were obtained.
¹H-NMR (270 MHz, CDCℓ₃) δ:
   8.84(1H,dd,J=7.92,1.32Hz), 7.69(1H,d,J=7.59Hz), 7.54(1H,t,J=7.92Hz), 7.40-7.46(5H,m), 7.11(1H,s), 5.26(2H,s), 3.74-3.76(4H,m), 3.65(3H,s), 3.51-3.60(4H,m), 2.77(3H,s), 2.08-2.24(2H,m).

### Example 12

### 1-(1-Methoxy-4-methyl-5-isoquinolinesulfonyl)hexahydro-1H-1,4-diazepine

To a solution of 4-benzyloxycarbonyl-1-(1-methoxy-4-methyl-5-isoquinolinesulfonyl)hexahydro-1H- 1,4-diazepine (89.9 mg) in methanol (2.0 mℓ), 20% palladium hydroxide-carbon (93.8 mg) was added, followed by stirring for 2 hours under a hydrogen gas atmosphere of room temperature and atmospheric pressure. The reaction mixture was filtered and the filtrate was concentrated under reduced pressure. The resulting residue was purified by chromatography on a silica gel column (chloroform:methanol = 4:1), whereby 58.1 mg of the target compound were obtained.
¹H-NMR (270 MHz, d₆-DMSO) δ:
   8.67(1H,d,J=7.92Hz), 8.01(1H,d,J=7.59Hz), 7.67(1H,t,J=7.58Hz), 7.57(1H,s), 3.81(2H,brs), 3.66(2H,t,J=5.61Hz), 3.56(3H,s), 3.24(4H,m), 2.65(3H,s), 2.17(2H,m).
Mass (FAB) m/z for C₁₆H₂₁N₃O₃S: 336 (M+H⁺)

### Example 13

### 1-(4-Methyl-1-propoxy-5-isoquinolinesulfonyl)hexahydro-1H-1,4-diazepine

Using n-propyl bromide, treatments were conducted as in Examples 11 and 12, whereby 38.3 mg of the target compound were obtained.
¹H-NMR (270 MHz, d₆-DMSO) δ:
   9.40(1H,brs), 8.72(1H,d,J=7.91Hz), 8.04(1H,d,J=7.59Hz), 7.71(1H,t,J=7.92Hz), 7.60(1H,s), 4.00(2H,t,J=7.25Hz), 3.85-3.86(2H,m), 3.69(2H,t,J=5.93Hz), 3.33-3.42(4H,m), 2.69(3H,s), 2.22(2H,m), 1.75-1.83(2H,m), 0.97(3H,m).
Mass (FAB) m/z for C₁₈H₂₅N₃O₃S: 364 (M+H⁺)

### Example 14

### 1-(1-n-Hexyloxy-4-methyl-5-isoquinolinesulfonyl)hexahydro-1H-1,4-diazepine

Using n-hexyl bromide, treatments were conducted as in Examples 11 and 12, whereby 52.0 mg of the target compound were obtained.
¹H-NMR (270 MHz, d₆-DMSO) δ:
   8.70(1H,d,J=7.92Hz), 8.01(1H,d,J=7.59Hz), 7.70(1H,t,J=7.92Hz), 7.58(1H,s), 4.01(2H,t,J=7.26Hz), 3.63-3.70(4H,m), 3.15(4H,m), 2.68(3H,s), 2.05(2H,brs), 1.75(2H,brs), 1.35(6H,brs), 0.92(3H,brs).
Mass (FAB) m/z for C₂₁H₃₁N₃O₃S: 406 (M+H⁺)

### Example 15

### 1-(1-(2-Amino)ethoxy-4-methyl-5-isoquinolinesulfonyl)hexahydro-1H-1,4-diazepine

Using N-benzyloxycarbonyl-2-aminoethyl bromide, treatments were conducted as in Examples 11 and 12, whereby 76.8 mg of the target compound were obtained.
¹H-NMR (270 MHz, CD₃OD) δ:
   8.62(1H,d,J=7.59Hz), 7.84(1H,d,J=7.25Hz), 7.54-7.62(1H,m), 7.31(1H,m), 4.13-4.21(2H,m), 3.57-3.66(5H,m), 3.02-3.07(5H,m), 2.68(3H,s), 1.91-1.99(2H,m).
Mass (FAB) m/z for C₁₇H₂₄N₄O₃S: 365 (M+H⁺)

### Example 16

### 1-(1-(6-Amino)hexyloxy-4-methyl-5-isoquinolinesulfonyl)hexahydro-1H-1,4-diazepine

Using N-benzyloxycarbonyl-6-aminohexyl bromide, treatments were conducted as in Examples 11 and 12, whereby 104.3 mg of the target compound were obtained.
¹H-NMR (270 MHz, CDCℓ₃) δ:
   8.77(1H,d,J=7.92Hz), 7.74(1H,d,J=7.92Hz), 7.49(1H,t,J=7.92Hz), 7.03(1H,s), 3.97(2H,t,J=7.26Hz), 3.60(2H,t,J=6.27Hz), 3.55(2H,t,J=5.28Hz), 3.07(4H,t,J=5.61Hz), 2.67-2.73(4H,m), 2.43(3H,brs), 1.92-2.00(2H,m), 1.78-1.81(2H,m), 1.40(4H,m).
Mass (FAB) m/z for C₂₁H₃₂N₄O₃S: 421 (M+H⁺)

### Example 17

### 1-(1-n-Butyloxy-4-methyl-5-isoquinolinesulfonyl)hexahydro-1H-1,4-diazepine

Using n-butyl bromide, treatments were conducted as in Examples 11 and 12, whereby 29.6 mg of the target compound were obtained.
¹H-NMR (270 MHz, CDCℓ₃) δ:
   8.81(1H,d,J=7.92Hz), 7.67(1H,d,J=7.91Hz), 7.51(1H,t,J=7.91Hz), 7.03(1H,s), 3.91-4.01(4H,m), 3.78(2H,m), 3.44-3.53(4H,m), 2.68(3H,s), 2.41(2H,m), 1.76(2H,m), 1.39-1.42(2H,m), 0.97(3H,t,J=6.92Hz).
Mass (FAB) m/z for C₁₉H₂₇N₃O₃S: 378 (M+H⁺)

### Example 18

### 1-(4-Methyl-1-n-pentyloxy-5-isoquinolinesulfonyl)hexahydro-1H-1,4-diazepine

Using n-pentyl bromide, treatments were conducted as in Examples 11 and 12, whereby 37.7 mg of the target compound were obtained.
¹H-NMR (270 MHz, CDCℓ₃) δ:
   8.81(1H,dd,J=8.25,1.32Hz), 7.68(1H,d,J=6.26Hz), 7.51(1H,t,J=7.91Hz), 7.03(1H,s), 3.91-4.00(4H,m), 3.78(2H,t,J=6.60Hz), 3.44-3.52(4H,m), 2.69(3H,s), 2.41(2H,m), 1.75-1.77(2H,m), 1.33-1.39(4H,m), 0.91(3H,t,J=6.93Hz).
Mass (FAB) m/z for C₂₀H₂₉N₃O₃S: 392 (M+H⁺)

### Example 19

### 1-(1-n-Heptyloxy-4-methyl-5-isoquinolinesulfonyl)hexahydro-1H-1,4-diazepine

Using heptyl bromide, treatments were conducted as in Examples 11 and 12, whereby 34.8 mg of the target compound were obtained.
¹H-NMR (270 MHz, CDCℓ₃) δ:
   8.81(1H,dd,J=7.92,1.32Hz), 7.68(1H,d,J=6.26Hz), 7.51(1H,t,J=7.91Hz), 7.03(1H,s), 3.89-3.99(4H,m), 3.77(2H,t,J=6.60Hz), 3.43-3.49(4H,m), 2.69(3H,s), 2.39(2H,m), 1.77(2H,m), 1.18-1.34(8H,m), 0.88(3H,t,J=6.60Hz).
Mass (FAB) m/z for C₂₂H₃₃N₃O₃S: 420 (M+H⁺)

### Example 20

### 1-(4-Methyl-1-n-octyloxy-5-isoquinolinesulfonyl)hexahydro-1H-1,4-diazepine

Using n-octyl bromide, treatments were conducted as in Examples 11 and 12, whereby 41.1 mg of the target compound were obtained.
¹H-NMR (270 MHz, CDCℓ₃) δ:
   8.80(1H,dd,J=7.92,0.99Hz), 7.70(1H,dd,J=7.59,1.32Hz), 7.51(1H,t,J=7.75Hz), 7.03(1H,s), 3.94-3.99(4H,m), 3.71-3.76(2H,t,J=6.60Hz), 3.39-3.46(4H,m), 2.69(3H,s), 2.32-2.34(2H,m), 1.74-1.79(2H,m), 1.20-1.44(8H,m), 0.85-0.90(3H,m).
Mass (FAB) m/z for C₂₃H₃₅N₃O₃S: 434 (M+H⁺)

### Example 21

### 1-(4-Methyl-1-n-octadecyloxy-5-isoquinolinesulfonyl)hexahydro-1H-1,4-diazepine

Using n-octadecyl bromide, treatments were conducted as in Examples 11 and 12, whereby 49.2 mg of the target compound were obtained.
¹H-NMR (270 MHz, CDCℓ₃) δ:
   8.78(1H,dd,J=7.92,1.32Hz), 7.72(1H,d,J=6.27Hz), 7.49(1H,t,J=7.92Hz), 7.02(1H,s), 3.96(2H,t,J=7.43Hz), 3.62-3.64(4H,m), 3.16-3.18(4H,m), 2.72(3H,s), 2.06-2.08(2H,m), 1.76(2H,m), 1.25-1.33(8H,m), 0.88(3H,m).
Mass (FAB) m/z for C₃₃H₅₅N₃O₃S: 574 (M+H⁺)

### Example 22

### 1-(1-Isopropyloxy-4-methyl-5-isoquinolinesulfonyl)hexahydro-1H-1,4-diazepine

Using isopropyl bromide, treatments were conducted as in Examples 11 and 12, whereby 7.5 mg of the target compound were obtained.
¹H-NMR (270 MHz, CDCℓ₃) δ:
   8.81(1H,d,J=6.93Hz), 7.69(1H,d,J=6.93Hz), 7.49(1H,t,J=6.93Hz), 7.07(1H,s), 5.30(1H,m), 3.68-3.71(4H,m), 3.26(4H,m), 2.74(3H,s), 2.17(2H,m), 1.40(6H,d,J=6.93Hz).
Mass (FAB) m/z for C₁₈H₂₅N₃O₃S: 364 (M+H⁺)

### Example 23

### 1-(4-Methyl-1-(2-methyl)propyloxy-5-isoquinolinesulfonyl)hexahydro-1H-1,4-diazepine

Using isobutyl bromide, treatments were conducted as in Examples 11 and 12, whereby 14.6 mg of the target compound were obtained.
¹H-NMR (270 MHz, CDCℓ₃) δ:
   8.81(1H,d,J=7.59Hz), 7.69(1H,d,J=7.91Hz), 7.51(1H,t,J=7.91Hz), 7.00(1H,s), 3.76-3.85(6H,m), 3.39-3.46(4H,m), 2.69(3H,s), 2.35(2H,m), 2.17-2.22(1H,m), 0.97(6H,d,J=6.60Hz).
Mass (FAB) m/z for C₁₉H₂₇N₃O₃S: 378 (M+H⁺)

### Example 24

### 1-(4-Methyl-1-(1-methyl)propyloxy-5-isoquinolinesulfonyl)hexahydro-1H-1,4-diazepine

Using sec-butyl bromide, treatments were conducted as in Examples 11 and 12, whereby 4.9 mg of the target compound were obtained.
¹H-NMR (270 MHz, CDCℓ₃) δ:
   8.80(1H,d,J=7.92Hz), 7.72(1H,d,J=6.27Hz), 7.49(1H,t,J=7.92Hz), 6.99(1H,s), 5.10(1H,m), 3.61-3.65(4H,m), 3.05-3.15(4H,m), 2.75(3H,s), 1.97-2.17(2H,m), 1.38(3H,d,J=6.60Hz), 1.16-1.25(2H,m), 0.89(3H,t,J=7.26Hz).
Mass (FAB) m/z for C₁₉H₂₇N₃O₃S: 378 (M+H⁺)

### Example 25

### 1-(1-(2-Ethyl)hexyloxy-4-methyl-5-isoquinolinesulfonyl)hexahydro-1H-1,4-diazepine

Using 2-ethylhexyl bromide, treatments were conducted as in Examples 11 and 12, whereby 8.1 mg of the target compound were obtained.
¹H-NMR (270 MHz, CDCℓ₃) δ:
   8.79(1H,d,J=7.92Hz), 7.72(1H,d,J=7.59Hz), 7.49(1H,t,J=7.92Hz), 6.98(1H,s), 3.88(2H,d,J=7.25Hz), 3.63-3.65(4H,m), 3.17-3.31(4H,m), 2.72(3H,s), 2.05-2.09(2H,m), 1.89(1H,m), 1.26-1.38(8H,m), 0.89-0.94(6H,m).
Mass (FAB) m/z for C₂₃H₃₅N₃O₃S: 434 (M+H⁺)

### Example 26

### 1-(1-Cyclohexylmethyloxy-4-methyl-5-isoquinolinesulfonyl)hexahydro-1H-1,4-diazepine

Using cyclohexylmethyl bromide, treatments were conducted as in Examples 11 and 12, whereby 15.7 mg of the target compound were obtained.
¹H-NMR (270 MHz, CDCℓ₃) δ:
   8.80(1H,d,J=7.59Hz), 7.69-7.73(1H,m), 7.52-7.56(1H,m), 6.99(1H,s), 4.16-4.28(2H,m), 3.48-3.88(8H,m), 2.69(3H,s), 2.26-2.42(2H,m), 0.87-1.81(11H,m).
Mass (FAB) m/z for C₂₂H₃₁N₃O₃S: 418 (M+H⁺)

### Example 27

### 1-(4-Methyl-1-(4-methyl)pentyloxy-5-isoquinolinesulfonyl)hexahydro-1H-1,4-diazepine

Using 4-methylpentyl bromide, treatments were conducted as in Examples 11 and 12, whereby 51.5 mg of the target compound were obtained.
¹H-NMR (270 MHz, CDCℓ₃) δ:
   8.81(1H,brs), 7.71(1H,brs), 7.53(1H,brs), 7.03(1H,s), 3.60-3.95(10H,m), 2.67(3H,s), 2.47(2H,brs), 1.77(2H,brs), 1.59-1.61(1H,m), 1.26(2H,m), 0.89(6H,d,J=6.60Hz).
Mass (FAB) m/z for C₂₁H₃₁N₃O₃S: 406 (M+H⁺)

### Example 28

### 1-(1-Hexyloxy-5-isoquinolinesulfonyl)hexahydro-1H-1,4-diazepine

Using 5-isoquinolinesulfonyl chloride and n-hexyl bromide, treatments were conducted as in Examples 7, 8, 9, 10, 11 and 12, whereby 45.0 mg of the target compound were obtained.
¹H-NMR (270 MHz, CDCℓ₃) δ:
   8.71(1H,d,J=7.92Hz), 8.17(1H,d,J=7.59Hz), 7.54(1H,t,J=7.92Hz), 7.23(1H,d,J=7.91Hz), 7.17(1H,d,J=7.92Hz), 4.00(2H,t,J=7.43Hz), 3.62-3.81(2H,m), 3.55(2H,t,J=6.27Hz), 3.22-3.52(4H,m), 2.17-2.34(2H,m), 1.68-1.84(2H,m), 1.20-1.47(6H,m), 0.79-0.98(3H,m).
Mass (FAB) m/z for C₂₀H₂₉N₃O₃S: 392 (M+H⁺)

### Example 29

### 1-(4-Methyl-5-isoquinolinesulfonyl)piperazine

Using piperazine, treatments were conducted as in Example 7, whereby 528.9 mg of the target compound were obtained.
¹H-NMR (270 MHz, CDCℓ₃) δ:
   9.15(1H,s), 8.55(1H,s), 8.18(2H,d,J=7.59Hz), 7.61(1H,t,J=7.92Hz), 3.45-3.49(4H,m), 3.08(3H,s), 2.99-3.02(4H,m).
Mass (FAB) m/z for C₁₄H₁₇N₃O₂S: 292 (M+H⁺)

### Example 30

### 1-(4-Methyl-5-isoquinolinesulfonyl)-2,5-dimethylpiperazine

Using 2,5-dimethylpiperazine, treatments were conducted as in Example 7, whereby 150.0 mg of the target compound were obtained.
¹H-NMR (270 MHz, CDCℓ₃) δ:
   9.12(1H,s), 8.54(1H,s), 8.28(1H,m), 8.12(1H,m), 7.58(1H,t,J=7.74Hz), 4.02-4.11(1H,m), 3.78-3.90(1H,m), 3.22-3.48(2H,m), 2.94-3.02(1H,m), 1.31(6H,t,J=6.26Hz).
Mass (FAB) m/z for C₁₆H₂₁N₃O₂S: 320 (M+H⁺)

### Example 31

### 1-(5-Isoquinolinesulfonyl)-6-methylhexahydro-1H-1,4-diazepine·dihydrochloride

Using 6-methylhexahydro-1H-1,4-diazepine synthesized in accordance with U.S. Patent No. 3,040,029 and 5-isoquinolinesulfonyl chloride, treatments were conducted as in Example 7, whereby 100.5 mg of the target compound were obtained.
¹H-NMR (270 MHz, d₆-DMSO) δ:
   9.62(1H,s), 8.78(1H,d,J=6.27Hz), 8.57(1H,d,J=8.24Hz), 8.45(1H,d,J=6.27Hz), 8.42(1H,d,J=7.58Hz), 7.94(1H,t,J=7.90Hz), 3.60-4.00(3H,m), 3.30-3.45(1H,m), 3.10-3.30(3H,m), 2.90-3.10(1H,m), 2.20-2.40(1H,brs), 0.97(3H,d,J=6.90Hz).

### Example 32

### 1-(4-Methyl-5-isoquinolinesulfonyl)-6-methylhexahydro-1H-1,4-diazepine·dihydrochloride

Using 6-methylhexahydro-1H-1,4-diazepine, treatments were conducted as in Example 7, whereby 95.5 mg of the target compound were obtained.
¹H-NMR (270 MHz, d₆-DMSO) δ:
   9.52(1H,brs), 8.63(1H,s), 8.56(1H,d,J=7.90Hz), 8.16(1H,d,J=7.90Hz), 7.89(1H,t,J=7.92Hz), 3.80-3.90(1H,m), 3.65-3.75(1H,m), 3.10-3.40(4H,m), 3.00(3H,s), 2.90-3.10(1H,m), 2.30-2.60(1H,m), 0.97(3H,d,J=6.93Hz).

### Example 33

### 1-(4-Methyl-5-isoquinolinesulfonyl)-5-methylhexahydro-1H-1,4-diazepine·dihydrochloride

Using as an amine the 5-methylhexahydro-1H-1,4-diazepine synthesized in Referential Example 5, treatments were conducted as in Example 7, whereby 65.3 mg of the target compound were obtained.
¹H-NMR (270 MHz, d₆-DMSO) δ:
   9.61(1H,s), 8.66(1H,s), 8.60(1H,d,J=7.60Hz), 8.30(1H,d,J=7.60Hz), 7.92(1H,t,J=7.62Hz), 3.75-4.00(2H,m), 3.60-3.75(2H,m), 3.45-3.60(1H,m), 3.30-3.45(1H,m), 3.15-3.30(1H,m), 3.00(3H,s), 2.05-2.25(2H,m), 1.38(3H,d,J=6.60Hz).

### Example 34

### 1-(1-Hydroxy-4-methyl-5-isoquinolinesulfonyl)-2,5-dimethylpiperazine

Using 2,5-dimethylpiperazine, treatments were conducted as in Examples 7, 8, 9, 10 and 12, whereby 20.0 mg of the target compound were obtained.
¹H-NMR (270 MHz, CDCℓ₃) δ:
   11.65(1H,brs), 9.38(1H,br), 8.59(1H,d,J=7.91Hz), 8.19(1H,d,J=6.93Hz), 7.63(1H,t,J=7.92Hz), 7.18(1H,brs), 4.04-4.12(1H,m), 3.81(1H,d,J=14.19Hz), 2.99-3.08(2H,br), 2.50(3H,s), 1.27(6H,t,J=6.26Hz).
Mass (FAB) m/z for C₁₆H₂₁N₃O₃S: 336 (M+H⁺)

### Example 35

### 1-(1-Hydroxy-4-methyl-5-isoquinolinesulfonyl)piperazine

Using piperazine, treatments were conducted as in Examples 7, 8, 9, 10 and 12, whereby 140.2 mg of the target compound were obtained.
¹H-NMR (270 MHz, d₆-DMSO) δ:
   11.76(1H,brs), 8.71(1H,d,J=6.92Hz), 8.20(1H,d,J=7.59Hz), 7.73(1H,t,J=7.92Hz), 7.29(1H,d,J=4.62Hz), 3.71(4H,m), 3.29(4H,m), 2.65(3H,s).
Mass (FAB) m/z for C₁₄H₁₇N₃O₃S: 308 (M+H⁺)

### Example 36

### 1-(1-Hydroxy-4-methyl-5-isoquinolinesulfonyl)-3-methylhexahydro-1H-1,4-diazepine·monohydrochloride

Using as an amine the 2-methylhexahydro-1H-1,4-diazepine synthesized in Referential Example 10, treatments were conducted as in Examples 7, 8, 9, 10 and 12, whereby 93.1 mg of the target compound were obtained.
¹H-NMR (270 MHz, d₆-DMSO) δ:
   11.57(1H,brs), 8.50(1H,dd,J=1.30,7.92Hz), 7.81(1H,dd,J=1.30,7.92Hz), 7.53(1H,t,J=7.92Hz), 7.10(1H,s), 3.50-3.70(2H,m), 3.10-3.50(4H,m), 2.85-3.00(1H,m), 2.43(3H,s), 1.85-2.00(2H,m), 1.08(3H,d,J=6.27Hz).

### Example 37

### 1-(1-Hydroxy-4-methyl-5-isoquinolinesulfonyl)-5-methylhexahydro-1H-1,4-diazepine·monohydrochloride

Using 5-methylhexahydro-1H-1,4-diazepine, treatments were conducted as in Examples 7, 8, 9, 10 and 12, whereby 88.8 mg of the target compound were obtained.
¹H-NMR (270 MHz, d₆-DMSO) δ:
   11.73(1H,brs), 9.28(1H,brs), 8.67(1H,dd,J=7.92,1.60Hz), 8.04(1H,dd,J=7.92,1.60Hz), 7.69(1H,t,J=7.92Hz), 7.27(1H,brs), 3.80-3.90(2H,m), 3.60-3.76(2H,m), 3.50-3.60(1H,m), 3.20-3.50(2H,m), 2.66(3H,s), 2.10-2.20(2H,m), 1.43(3H,d,J=6.60Hz).

### Example 38

### 4-Acetyl-1-(1-chloro-4-methyl-5-isoquinolinesulfonyl)hexahydro-1H-1,4-diazepine

To a solution of 4-acetyl-1-(4-methyl-2-oxy-5-isoquinolinesulfonyl)hexahydro-1H-1,4-diazepine (1.48 g) in dimethylformamide (2.0 mℓ), toluene (4.0 mℓ) was added, followed by stirring for 10 minutes. Phosphorus oxychloride (06 mℓ) was added dropwise to the reaction mixture under ice cooling. The resulting mixture was stirred at the same temperature for 10 minutes and further at 50°C for 1 hour. The solvents were distilled off under reduced pressure and, to the resultant residue, ethyl acetate (150 mℓ) was added. The thus-obtained mixture was washed first with water (100 mℓ x 2) and then with a saturated aqueous solution of sodium chloride (100 mℓ), and was then dried over anhydrous magnesium sulfate. The solvent was distilled off under reduced pressure, and the resultant residue was purified by chromatography on a silica gel column (ethyl acetate:methanol = 30:1), whereby 0.61 g of the target compound was obtained.
¹H-NMR (270 MHz, CDCℓ₃) δ:
   8.66(1H,d,J=8.58Hz), 8.27(1H,s), 7.81(1H,dd,J=9.90,7.59Hz), 7.68(1H,d,J=7.91Hz), 3.70-3.81(4H,m), 3.55-3.66(4H,m), 3.02(3H,s), 2.19(3H,s), 2.05-2.18(2H,m).

### Example 39

### 4-Acetyl-1-(4-methyl-1-methylthio-5-isoquinolinesulfonyl)hexahydro-1H-1,4-diazepine

To a solution of 4-acetyl-1-(1-chloro-4-methyl-5-isoquinolinesulfonyl)hexahydro-1H-1,4-diazepine (90.6 mg) in dimethylformamide (0.5 mℓ), a 15% aqueous solution of sodium thiomethoxide (0.2 mℓ) was added, followed by stirring at 80°C for 14 hours. Ethyl acetate (100 mℓ) was added to the reaction mixture. The resultant mixture was washed first with water (50 mℓ x 2) and then with a saturated aqueous solution of sodium chloride (50 mℓ), and was then dried over anhydrous magnesium sulfate. The solvent was then distilled off under reduced pressure. The resulting residue was purified by chromatography on a silica gel column (ethyl acetate), whereby 83.6 g of the target compound were obtained.
¹H-NMR (270 MHz, CDCℓ₃) δ:
   8.47(1H,d,J=8.58Hz), 8.32(1H,s), 7.71(1H,t,J=7.59Hz), 7.48-7.55(1H,m), 3.65-3.79(4H,m), 3.50-3.59(4H,m), 2.95(3H,s), 2.67(3H,s), 2.16(3H,s), 2.03-2.15(2H,m).

### Example 40

### 1-(4-Methyl-1-methylthio-5-isoquinolinesulfonyl)hexahydro-1H-1,4-diazepine

A suspension of 4-acetyl-1-(4-methyl-1-methylthio-5-isoquinolinesulfonyl)hexahydro-1H-1,4-diazepine (83.6 mg) in a 1 N aqueous solution of hydrochloric acid (2.0 mℓ) was heated under reflux at 100°C for 12 hours. The solvent was distilled off under reduced pressure. The resulting residue was purified by chromatography on a silica gel column (chloroform:methanol = 10:1), whereby 38.4 mg of the target compound were obtained.
¹H-NMR (270 MHz, CD₃OD) δ:
   8.47(1H,dd,J=8.42,1.32Hz), 8.26(1H,d,J=0.99Hz), 7.93(1H,dd,J=7.59,1.32Hz), 7.65(1H,t,J=8.25Hz), 3.63-3.74(4H,m), 3.18-3.24(4H,m), 2.91(3H,s), 2.64(3H,s), 2.03-2.12(2H,m).
Mass (FAB) m/z for C₁₆H₂₁N₃O₂S₂: 352 (M+H⁺)

### Example 41

### 1-(4-Methyl-1-N-methylamino-5-isoquinolinesulfonyl)hexahydro-1H-1,4-diazepine

Using methylamine, treatments were conducted as in Examples 39 and 40, whereby 10.6 mg of the target compound were obtained.
¹H-NMR (270 MHz, CD₃OD) δ:
   8.37(1H,dd,J=8.42,1.32Hz), 7.88(1H,dd,J=7.59,1.32Hz), 7.81(1H,d,J=0.66Hz), 7.58(1H,t,J=8.25Hz), 3.86(2H,t,J=5.61Hz), 3.70(2H,t,J=6.27Hz), 3.39-3.47(4H,m), 3.03(3H,s), 2.77(3H,s), 2.17-2.27(2H,m).
Mass (FAB) m/z for C₁₆H₂₂N₄O₂S: 335 (M+H⁺)

### Example 42

### 1-(1-N,N-dimethylamino-4-methyl-5-isoquinolinesulfonyl)hexahydro-1H-1,4-diazepine

Using dimethylamine, treatments were conducted as in Examples 39 and 40, whereby 4.4 mg of the target compound were obtained.
¹H-NMR (270 MHz, CD₃OD) δ:
   8.46(1H,dd,J=8.57,1.32Hz), 7.96(1H,d,J=0.66Hz), 7.89(1H,dd,J=7.43,1.32Hz), 7.59(1H,dd,J=8.25,7.59Hz), 3.62(4H,t,J=5.94Hz), 3.04-3.10(4H,m), 3.02(6H,s), 2.86(3H,s), 1.94-2.03(2H,m).
Mass (FAB) m/z for C₁₇H₂₄N₄O₂S: 349 (M+H⁺)

### Example 43

### 1-(1-Amino-4-methyl-5-isoquinolinesulfonyl)hexahydro-1H-1,4-diazepine

Using aqueous ammonia, treatments were conducted as in Examples 39 and 40, whereby 8.5 mg of the target compound were obtained.
¹H-NMR (270 MHz, CDCℓ₃) δ:
   8.10(1H,dd,J=8.25,0.99Hz), 7.79-7.81(2H,m), 7.52(1H,t,J=8.24Hz), 3.56-3.67(4H,m), 3.14-3.20(4H,m), 2.86(3H,s), 2.03-2.08(2H,m).
Mass (FAB) m/z for C₁₅H₂₀N₄O₂S: 321 (M+H⁺)

### Example 44

### 1-(1-n-Hexylthio-4-methyl-5-isoquinolinesulfonyl)hexahydro-1H-1,4-diazepine

Using n-hexylmercaptan, treatments were conducted as in Examples 39 and 40, whereby 5.6 mg of the target compound were obtained.
¹H-NMR (270 MHz, CDCℓ₃) δ:
   8.55(1H,d,J=7.26Hz), 8.32(1H,s), 7.78(1H,d,J=6.60Hz), 7.54(1H,t,J=8.08Hz), 3.68-3.70(4H,m), 3.33(2H,t,J=7.26Hz), 3.22(4H,m), 2.97(3H,s), 2.11-2.13(2H,m), 1.70-1.81(2H,m), 1.46-1.48(2H,m), 1.25-1.35(4H,m), 0.89-0.92(3H,m).
Mass (FAB) m/z for C₂₁H₃₁N₃O₂S₂: 422 (M+H⁺)

### Example 45

### 1-(1-n-Hexylamino-4-methyl-5-isoquinolinesulfonyl)hexahydro-1H-1,4-diazepine

Using n-hexylamine, treatments were conducted as in Examples 39 and 40, whereby 69.9 mg of the target compound were obtained.
¹H-NMR (270 MHz, CDCℓ₃) δ:
   8.02(1H,d,J=7.26Hz), 8.00(1H,s), 7.73(1H,d,J=7.26Hz), 7.43(1H,t,J=8.09Hz), 5.10(1H,brs), 3.50-3.63(6H,m), 3.07-3.11(4H,m), 2.85(3H,s), 2.11(2H,brs), 1.91-2.00(2H,m), 1.65-1.76(2H,m), 1.32-1.43(4H,m), 0.90-0.92(3H,m).
Mass (FAB) m/z for C₂₁H₃₂N₄O₂S: 405 (M+H⁺)

### Example 46

### 1-(1-Allyloxy-4-methyl-5-isoquinolinesulfonyl)hexahydro-1H-1,4-diazepine

Using 4-acetyl-1-(1-hydroxy-4-methyl-5-isoquinolinesulfonyl)hexahydro-1H-1,4-diazepine and allyl bromide, treatments were conducted as in Example 11 and then as in Example 40, whereby 61.8 mg of the target compound were obtained.
¹H-NMR (270 MHz, CDCℓ₃) δ:
   8.82(1H,brs), 7.70(1H,brs), 7.54(1H,brs), 7.02(1H,s), 5.95-5.97(1H,m), 5.30(1H,d,J=9.24Hz), 5.25(1H,d,J=15.83Hz), 4.62(2H,d,J=4.95Hz), 3.56-3.95(8H,m), 2.68(3H,s), 2.46(2H,brs).
Mass (FAB) m/z for C₁₈H₂₃N₃O₃S: 362 (M+H⁺)

### Example 47

### 1-(1-Benzyloxy-4-methyl-5-isoquinolinesulfonyl)hexahydro-1H-1,4-diazepine

Using 4-acetyl-1-(1-hydroxy-4-methyl-5-isoquinolinesulfonyl)hexahydro-1H-1,4-diazepine and benzyl bromide, treatments were conducted as in Example 11 and then as in Example 40, whereby 46.4 mg of the target compound were obtained.
¹H-NMR (270 MHz, CDCℓ₃) δ:
   8.85(1H,brs), 7.65(2H,brs), 7.33(5H,brs), 7.06(1H,s), 5.19(2H,brs), 3.40-4.20(8H,m), 2.64(3H,s), 2.50-2.60(2H,m).
Mass (FAB) m/z for C₂₂H₂₅N₃O₃S: 412 (M+H⁺)

### Example 48

### 1-(4-Methyl-1-pentafluorophenylmethyloxy-5-isoquinolinesulfonyl)hexahydro-1H-1,4-diazepine

Using 4-acetyl-1-(1-hydroxy-4-methyl-5-isoquinolinesulfonyl)hexahydro-1H-1,4-diazepine and pentafluorophenylmethyl bromide, treatments were conducted as in Example 11 and then as in Example 40, whereby 51.2 mg of the target compound were obtained.
¹H-NMR (270 MHz, CDCℓ₃) δ:
   8.75(1H,brs), 7.40-7.80(2H,brs), 7.13(1H,brs), 5.22(2H,brs), 3.40-3.90(8H,m), 2.70(3H,s), 2.60-2.70(2H,m).
Mass (FAB) m/z for C₂₂H₂₀F₅N₃O₃S: 502 (M+H⁺)

### Example 49

### 4-Benzyloxycarbonyl-1-(4-methyl-5-isoquinolinesulfonyl)-2-methylpiperazine

To a solution of 1-benzyloxycarbonyl-3-methylpiperazine (5.09 g) in chloroform (150 mℓ), a solution of triethylamine (3.0 mℓ) and 4-methyl-5-isoquinolinesulfonyl chloride (6.05 g) in chloroform (50 mℓ) was gradually added dropwise. After completion of the dropwise addition, the resultant mixture was continuously stirred at 60°C for 20 hours. The reaction mixture was washed first with water (100 mℓ x 2) and then with a saturate aqueous solution of sodium chloride (100 mℓ), and was then dried over anhydrous magnesium sulfate. The solvent was then distilled off under reduced pressure. The resulting residue was purified by chromatography on a silica gel column (chloroform), whereby 3.95 g of the target compound were obtained.
¹H-NMR (270 MHz, CDCℓ₃) δ:
   9.15(1H,s), 8.54(1H,s), 8.37(1H,dd,J=7.92,1.32Hz), 8.17(1H,dd,J=7.92,1.32Hz), 7.61(1H,t,J=7.59Hz), 7.36(5H,m), 5.17(2H,dd,J=14.85,12.21Hz), 4.05-4.18(3H,m), 3.33-3.55(3H,m), 3.04(3H,s), 1.34(3H,d,J=6.60Hz).

### Example 50

### 1-(1-Hydroxy-4-methyl-5-isoquinolinesulfonyl)2-methylpiperazine

Using 4-benzyloxycarbonyl-1-(4-methyl-5-isoquinolinesulfonyl)-2-methylpiperazine, treatments were conducted as in Examples 9, 10 and 12, whereby 143.9 mg of the target compound were obtained.
¹H-NMR (270 MHz, d₆-DMSO) δ:
   8.69(1H,d,J=7.92Hz), 8.38(1H,d,J=6.60Hz), 7.74(1H,t,J=7.92Hz), 7.27(1H,brs), 3.26-3.46(7H,m), 2.63(3H,s), 1.49(3H,d,J=6.93Hz).
Mass (FAB) m/z for C₁₅H₁₉N₃O₃S: 322 (M+H⁺)

### Example 51

### 1-(1-Hydroxy-4-methyl-5-isoquinolinesulfonyl)2-methylhexahydro-1H-1,4-diazepine·monohydrochloride

Using 4-methyl-5-isoquinolinesulfonyl chloride and 1-benzyloxycarbonyl-3-methylhexahydro-1H-1,4-diazepine, treatments were conducted as in Examples 49, 9, 10 and 12, whereby 75.9 mg of the target compound were obtained.
¹H-NMR (270 MHz, d₆-DMSO) δ:
   11.79(1H,brd), 8.72(1H,dd,J=7.60,1.32Hz), 8.14(1H,dd,J=7.60,1.32Hz), 7.75(1H,t,J=7.60Hz), 7.31(1H,d,J=6.00Hz), 4.45-4.60(1H,m), 3.55-3.90(2H,m), 3.30-3.50(3H,m), 3.10-3.25(1H,m), 2.70(3H,s), 2.10-2.25(2H,m), 1.30(3H,d,J=6.60Hz).

### Example 52

### 1-(1-Hydroxy-4-methyl-5-isoquinolinesulfonyl)-7-methylhexahydro-1H-1,4-diazepine·monohydrochloride

Using the 1-benzyloxycarbonyl-5-methylhexahydro-1H-1,4-diazepine synthesized in Referential Example 6 and 4-methyl-isoquinolinesulfonyl chloride, treatments were conducted as in Examples 49, 9, 10 and 12, whereby 89.3 mg of the target compound were obtained.
¹H-NMR (270 MHz, d₆-DMSO) δ:
   11.70(1H,brs), 9.10(1H,brs), 8.67(1H,dd,J=7.90,1.30Hz), 8.19(1H,dd,J=7.90,1.30Hz), 7.70(1H,t,J=7.90Hz), 7.27(1H,brs), 4.15-4.35(1H,m), 3.70-3.95(2H,m), 3.40-3.60(2H,m), 3.00-3.20(2H,m), 2.66(3H,s), 2.30-2.45(1H,m), 2.00-2.20(1H,m), 1.19(3H,d,J=6.30Hz).

### Example 53

### 1-(1-n-Hexyloxy-4-methyl-5-isoquinolinesulfonyl)2-methylhexahydro-1H-1,4-diazepine

Using 1-benzyloxycarbonyl-3-methylhexahydro-1H-1,4-diazepine, treatments were conducted as in Examples 49, 9 and 10. Using n-hexyl bromide, treatments were then conducted as in Examples 11 and 12, whereby 72.0 mg of the target compound were obtained.
¹H-NMR (270 MHz, CDCℓ₃) δ:
   8.78(1H,d,J=7.92Hz), 8.49(1H,d,J=7.92Hz), 7.48(1H,t,J=7.92Hz), 7.01(1H,s), 4.10-4.26(1H,m), 3.89-4.01(2H,m), 3.72-3.88(1H,m), 3.29-3.44(2H,m), 3.23(1H,dt,J=4.62,13.20Hz), 2.77-2.90(1H,m), 2.74(3H,s), 2.67(1H,dd,J=8.91,14.51Hz), 2.19-2.65(1H,m), 1.96-2.14(1H,m), 1.62-1.84(2H,m), 1.21(3H,d,J=5.39Hz), 1.18-1.43(6H,m), 0.79-0.96(3H,m).
Mass (FAB) m/z for C₂₂H₃₃N₃O₃S: 420 (M+H⁺)

### Example 54

### 1-(4-Fluoro-5-isoquinolinesulfonyl)-2-methylhexahydro-1H-1,4-diazepine

Using the 4-fluoro-5-isoquinolinesulfonyl chloride obtained in Referential Example 4 and the 1-t-butoxycarbonyl-3-methylhexahydro-1H-1,4-diazepine synthesized in Referential Example 11, treatments were conducted as in Example 1, whereby 20.0 mg of the target compound were obtained.
¹H-NMR (270 MHz, CDCℓ₃) δ:
   9.15(1H,s), 8.83(1H,d,J=7.59Hz), 8.57(1H,d,J=4.62Hz), 8.22(1H,d,J=8.58Hz), 7.73(1H,t,J=7.92Hz), 3.92-4.11(2H,m), 3.12-3.38(3H,m), 2.63-2.79(1H,m), 2.54(1H,dd,J=14.52,8.91Hz), 1.59-2.08(2H,m), 0.90(3H,d,J=6.60).
Mass (FAB) m/z for C₁₅H₁₈FN₃O₂S: 324 (M+H⁺)

### Example 55

### 1-(4-Fluoro-5-isoquinolinesulfonyl)hexahydro-1H-1,4-diazepine

Using 4-fluoro-5-isoquinolinesulfonyl chloride and hexahydro-1H-1,4-diazepine, treatments were conducted as in Example 7, whereby 120.0 mg of the target compound were obtained.
¹H-NMR (270 MHz, CDCℓ₃) δ:
   9.34(1H,s), 8.66(1H,d,J=4.62Hz), 8.50(1H,brd,J=9.24Hz), 8.23(1H,d,J=6.93Hz), 7.87(1H,t,J=7.59Hz), 3.68(1H,t,J=5.94Hz), 3.56(2H,t,J=5.94Hz), 3.47(2H,t,J=4.95Hz), 2.81-2.89(4H,m), 1.73-1.81(2H,m).
Mass (FAB) m/z for C₁₄H₁₆FN₃O₂S: 310 (M+H⁺)

### Example 56

### 1-(4-Fluoro-5-isoquinolinesulfonyl)-5-methylhexahydro-1H-1,4-diazepine

Using 4-fluoro-5-isoquinolinesulfonyl chloride and 5-methyl-hexahydro-1H-1,4-diazepine, treatments were conducted as in Example 7, whereby 120.0 mg of the target compound were obtained.
¹H-NMR (270 MHz, CDCℓ₃) δ:
   9.28(1H,brs), 8.66(1H,d,J=4.64Hz), 8.48(1H,d,J=9.24Hz), 8.24(1H,d,J=6.92Hz), 7.86(1H,t,J=5.94Hz), 3.76-3.98(2H,m), 3.63-3.74(2H,m), 3.46-3.58(1H,m), 3.34-3.43(1H,m), 3.14-3.32(1H,m), 2.06-2.14(2H,m), 1.35(3H,d,J=6.60Hz).
Mass (FAB) m/z for C₁₅H₁₈FN₃O₂S: 324 (M+H⁺)

### Example 57

### 1-(4-Fluoro-5-isoquinolinesulfonyl)-2,5-dimethylpiperazine

Using 4-fluoro-5-isoquinolinesulfonyl chloride and 2,5-dimethylpiperazine, treatments were conducted as in Example 7, whereby 190.0 mg of the target compound were obtained.
¹H-NMR (270 MHz, CDCℓ₃) δ:
   9.14(1H,brs), 8.55(2H,d,J=4.95Hz), 8.22(1H,d,J=7.92Hz), 7.72(1H,t,J=7.92Hz), 3.78-3.91(1H,m), 3.20-3.48(2H,m), 2.94-3.04(1H,m), 1.31(6H,t,J=6.24Hz).
Mass (FAB) m/z for C₁₅H₁₈FN₃O₂S: 324 (M+H⁺)

### Example 58

### 4-Benzyloxycarbonyl-1-(4-fluoro-2-oxy-5-isoquinolinesulfonyl)hexahydro-1H-1,4-diazepine

Using 1-(4-fluoro-5-isoquinolinesulfonyl)hexahydro-1H-1,4-diazepine, treatments were conducted as in Examples 8 and 9, whereby 2.40 g of the target compound were obtained.
¹H-NMR (270 MHz, CDCℓ₃) δ:
   8.71(1H,s), 8.26(1H,d,J=6.93Hz), 7.71-8.01(7H,m), 7.68(1H,d,J=7.92Hz), 7.36(5H,s), 5.15(2H,d,J=2.97Hz), 3.67(4H,d,J=6.27Hz), 3.46-3.55(4H,m), 2.09(2H,s), 2.00-2.05(2H,m).

### Example 59

### 4-Benzyloxycarbonyl-1-(4-fluoro-1-hydroxy-5-isoquinolinesulfonyl)hexahydro-1H-1,4-diazepine

4-Benzyloxycarbonyl-1-(4-fluoro-2-oxy-5-isoquinolinesulfonyl)hexahydro-1H-1,4-diazepine (1.80 g) was dissolved in tetrahydrofuran (3.0 mℓ), followed by the addition of trifluoroacetic anhydride (5.0 mℓ). The resultant mixture was stirred at 60°C for 1 hour, followed by the addition of water (10 mℓ). The reaction mixture so obtained was stirred at room temperature for 5 minutes. The reaction mixture was extracted with chloroform. The extract was washed with water and a saturated aqueous solution of sodium chloride, and was then dried over anhydrous magnesium sulfate. The solvent was then distilled off under reduced pressure. The resulting residue was purified by chromatography on a silica gel column, whereby 1.11 g of the target compound were obtained from fractions eluted with ethyl acetate.
¹H-NMR (270 MHz, CDCℓ₃) δ:
   8.67(1H,d,J=8.25Hz), 8.12(1H,t,J=8.57Hz), 7.63(1H,t,J=7.92Hz), 7.36(5H,s), 7.25-7.27(1H,m), 5.15(2H,d,J=3.30Hz), 3.66(2H,t,J=5.94Hz), 3.45-3.52(2H,m), 3.44(2H,t,J=5.94Hz), 1.98-2.03(2H,m).
Mass (FAB) m/z for C₂₂H₂₂FN₃O₅S: 460 (M+H⁺)

### Example 60

### 1-(4-Fluoro-1-hydroxy-5-isoquinolinesulfonyl)hexahydro-1H-1,4-diazepine

4-Benzyloxycarbonyl-1-(4-fluoro-1-hydroxy-5-isoquinolinesulfonyl)hexahydro-1H-1,4-diazepine (0.21 g) was dissolved in ethanol (2.0 mℓ), followed by the addition of 20% palladium hydroxide-carbon (0.2 g). The resultant mixture was stirred at room temperature under a hydrogen gas atmosphere. After completion of a reaction, the solvent was then distilled off under reduced pressure. The resulting residue was recrystallized from ethanol-hexane, whereby 80.0 mg of the target compound were obtained.
¹H-NMR (270 MHz, CDCℓ₃) δ:
   11.62(1H,brs), 9.02(1H,br), 8.56(1H,brd,J=4.94Hz), 8.12(1H,d,J=8.54Hz), 7.48-7.52(1H,m), 6.88(1H,t,J=7.58Hz), 3.81(2H,brt,J=5.94Hz), 3.62(2H,t,J=4.92Hz), 3.24-3.36(4H,m), 2.11-2.18(2H,m).
Mass (FAB) m/z for C₁₄H₁₆FN₃O₃S: 326 (M+H⁺)

### Example 61

### 1-(4-Fluoro-1-hydroxy-5-isoquinolinesulfonyl)-5-methylhexahydro-1H-1,4-diazepine

Using 1-(4-fluoro-5-isoquinolinesulfonyl)-5-methylhexahydro-1H-1,4-diazepine, treatments were conducted as in Examples 8, 9, 59 and 60, whereby 19.0 mg of the target compound were obtained.
¹H-NMR (270 MHz, CDCℓ₃) δ:
   9.28(1H,brs), 9.22(1H,brs), 8.53(1H,d,J=7.58Hz), 8.13(1H,d,J=8.54Hz), 7.70(1H,t,J=7.71Hz), 3.77(2H,t,J=5.96Hz), 3.58(2H,t,J=4.95Hz), 3.52-3.60(1H,m), 3.23-3.50(2H,m), 2.08-2.20(2H,m),1.42(3H,d,J=6.60Hz).
Mass (FAB) m/z for C₁₅H₁₈FN₃O₃S: 340 (M+H⁺)

### Example 62

### 1-(4-Fluoro-1-hydroxy-5-isoquinolinesulfonyl)-2,5-dimethylpiperazine

Using 1-(4-fluoro-5-isoquinolinesulfonyl)-2,5-dimethylpiperazine, treatments were conducted as in Examples 8, 9, 59 and 60, whereby 22.0 mg of the target compound were obtained.
¹H-NMR (270 MHz, CDCℓ₃) δ:
   9.23(1H,br), 8.54(1H,d,J=4.95Hz), 8.12(1H,d,J=8.54Hz), 6.82(1H,t,J=7.54Hz), 4.01-4.10(1H,m), 3.82(1H,d,J=14.22Hz), 3.46-3.52(2H,m), 2.96-3.06(1H,m), 1.28(6H,t,J=6.26Hz).
Mass (FAB) m/z for C₁₄H₁₆FN₃O₂S: 340 (M+H⁺)

### Example 63

### 4-Benzyloxycarbonyl-1-(4-fluoro-5-isoquinolinesulfonyl)-7-methylhexahydro-1H-1,4-diazepine

Using 4-fluoro-5-isoquinolinesulfonyl chloride and 1-bezyloxycarbonyl-5-methylhexahydro-1H-1,4-diazepine, treatments were conducted as in Example 49, whereby 2.6 g of the target compound were obtained.
¹H-NMR (270 MHz, CDCℓ₃) δ:
   9.14(1H,s), 8.53-8.58(2H,m), 8.23(1H,d,J=7.92Hz), 7.72(1H,t,J=7.92Hz), 7.34(5H,s), 5.12(2H,d,J=4.95Hz), 4.03-4.17(1H,m), 3.82-3.98(3H,m), 3.21-3.45(3H,m), 2.06-1.73(1H,m), 1.65-1.73(1H,m), 0.99(3H,d,J=6.59Hz).
Mass (FAB) m/z for C₂₃H₂₄FN₃O₄S: 458 (M+H⁺)

### Example 64

### 1-(4-Fluoro-5-isoquinolinesulfonyl)-7-methylhexahydro-1H-1,4-diazepine

Using 4-benzyloxycarbonyl-1-(4-fluoro-5-isoquinolinesulfonyl)-7-methylhexahydro-1H-1,4-diazepine, treatments were conducted as in Example 60, whereby 180.0 mg of the target compound were obtained.
¹H-NMR (270 MHz, CDCℓ₃) δ:
   9.17(1H,brs), 8.82(1H,d,J=7.59Hz), 8.56(1H,d,J=4.62Hz), 8.22(1H,d,J=8.58Hz), 7.72(2H,t,J=7.92Hz), 4.01-4.07(1H,m), 3.84(1H,t,J=17.15Hz), 3.58-3.72(1H,m), 2.90-3.00(1H,m), 2.71-2.89(1H,m), 2.25-2.31(1H,m), 1.91-2.05(1H,m), 0.89(3H,d,J=6.27Hz).
Mass (FAB) m/z for C₁₅H₁₈FN₃O₂S: 324 (M+H⁺)

### Example 65

### 1-(4-Fluoro-1-hydroxy-5-isoquinolinesulfonyl)-7-methylhexahydro-1H-1,4-diazepine

Using 4-benzyloxycarbonyl-1-(4-fluoro-5-isoquinolinesulfonyl)-7-methylhexahydro-1H-1,4-diazepine, treatments were conducted as in Examples 9, 60 and 61, whereby 55.0 mg of the target compound were obtained.
¹H-NMR (270 MHz, CDCℓ₃) δ:
   11.60(1H,brs), 9.12(1H,brs), 8.54(1H,d,J=4.96Hz), 8.12(1H,d,J=8.54Hz), 6.84(1H,t,J=7.54Hz), 4.03-4.10(1H,m), 3.71-3.86(1H,m), 3.46-3.66(1H,m), 2.87-3.02(1H,m), 2.24-2.32(1H,m), 1.88-2.02(1H,m), 0.88(3H,d,J=6.24Hz).
Mass (FAB) m/z for C₁₅H₁₈FN₃O₂S: 340 (M+H⁺)

### Example 66

### 1-(4-Methyl-8-isoquinolinesulfonyl)hexahydro-1H-1,4-diazepine

A solution of the 4-methyl-8-isoquinolinesulfonic acid·1/2 sulfate (8.55 g), which had been obtained in Referential Example 7, in thionyl chloride (30.0 mℓ) was heated under reflux at 90°C for 2.5 hours. The reaction mixture was concentrated under reduced pressure, and benzene (100 mℓ) was added to the residue. The resultant mixture was again concentrated under reduced pressure. Similar treatments were repeated three times, whereby a brown oil (8.58 g) was obtained. This oil (2.82 g) was dissolved in chloroform (30 mℓ), followed by the dropwise addition of a solution of hexahydro-1H-1,4-diazepine (4.12 g) in chloroform (150 mℓ) under ice cooling and stirring over 15 minutes. After completion of the dropwise addition, the mixture was stirred further at room temperature for 12 hours. The reaction mixture was washed first with water (100 mℓ x 2) and then with a saturated aqueous solution of sodium chloride (100 mℓ), and was then dried over anhydrous magnesium sulfate. The solvent was then distilled off under reduced pressure. The resulting residue was purified by chromatography on a silica gel column (chloroform:methanol = 8:1), whereby 2.17 g of the target compound were obtained.
¹H-NMR (270 MHz, CDCℓ₃) δ:
   9.97(1H,s), 8.53(1H,d,J=0.99Hz), 8.18-8.25(1H,m), 7.78(1H,dd,J=8.58,7.26Hz), 3.43-3.54(4H,m), 2.91-2.99(4H,m), 2.68(3H,s), 1.79-1.88(2H,m).

### Example 67

### 1-(4-Cyano-5-isoquinolinesulfonyl)-7-methylhexahydro-1H-1,4-diazepine

Using 4-cyano-5-isoquinolinesulfonyl chloride and 1-benzyloxycarbonyl-5-methylhexahydro-1H-1,4-diazepine, treatments were conducted as in Example 49 and then as in Example 60, whereby the target compound was obtained.
¹H-NMR (270 MHz, CDCℓ₃) δ:
   9.48(1H,s), 9.15(1H,s), 8.51(1H,dd,J=7.59,1.32Hz), 8.29(1H,dd,J=8.24,1.32Hz), 7.84(1H,t,J=7.91Hz), 4.30-4.39(1H,m), 3.84(1H,dt,J=15.51,2.97Hz), 3.35(1H,ddd,J=15.51,9.57,2.64Hz), 2.88-3.10(4H,m), 2.19-2.31(1H,m), 1.65-1.74(1H,m), 1.14(3H,d,J=6.59Hz).
Mass (FAB) m/z for C₁₆H₁₈N₄O₂S: 331 (M+H⁺)

### Example 68

### 1-(1,4-Dimethyl-7-isoquinolinesulfonyl)hexahydro-1H-1,4-diazepine

Using the 1,4-dimethylisoquinoline-7-sulfonyl chloride obtained in Referential Example 14 and hexahydro-1H-1,4-diazepine, the target compound was obtained.
¹H-NMR (270 MHz, CDCℓ₃) δ:
   8.62(1H,d,J=0.99Hz), 8.38(1H,s), 8.08(1H,d,J=8.91Hz), 8.01(1H,dd,J=8.91,1.65Hz), 3.38-3.48(4H,m), 2.94-3.09(4H,m), 2.99(3H,s), 2.69(3H,s), 1.87(2H,m).
Mass (FAB) m/z for C₁₆H₂₁N₃O₂S: 320 (M+H⁺)

### Test 1

### p24 Protein Production Inhibiting Effect (in vitro)

Quantitation of p24 protein was conducted by ELISA.

HIV-infected cells were lysed in a lysis buffer, and were poured in predetermined constant aliquots into the individual wells of a 96-well microplate. Subsequent to adsorption of an antigen, a solution of a biotinylated anti-p24 monoclonal antibody was added. A streptavidin-labeled peroxidase was added, followed by the addition of a substrate for staining. One, three and five days later or 2 and 4 days later, a terminating solution was added to terminate the reaction. Concerning each of the wells, the absorbance at 450 nm was measured to determine the content of p24 protein in the well. The results as calculated in terms of TCID₅₀/mℓ are shown in Tables 1-5.

**Table 1**

| | Day 0 (pg/mℓ) | Day 1 (pg/mℓ) | Day 2 (pg/mℓ) | Day 5 (pg/mℓ) |
|---|---|---|---|---|
| Control | 0 | 569542 | 881594 | 902226 |
| Example 6 (20 µg/m ) | 0 | 551489 | 645621 | 698489 |
| Example 51 (10 µg/m ) | 0 | 641752 | 624989 | 566963 |

**Table 2**

| | Day 0 (pg/m ) | Day 2 (pg/m ) | Day 4 (pg/m ) |
|---|---|---|---|
| Control | 0 | 46732 | 104484 |
| Example 14 (8 µg/mℓ) | 0 | 1022 | 0 |
| Example 29 (0.32 µg/mℓ) | 0 | 69877 | 69877 |
| Example 41 (0.8 µg/mℓ) | 0 | 82082 | 66977 |

**Table 3**

| | Day 0 (pg/mℓ) | Day 2 (pg/mℓ) | Day 4 (pg/mℓ) |
|---|---|---|---|
| Control | 0 | 104484 | 82079 |
| Example 55 (4 µg/mℓ) | 0 | 59487 | 59487 |

**Table 4**

| | Day 0 (pg/mℓ) | Day 2 (pg/mℓ) | Day 4 (pg/mℓ) |
|---|---|---|---|
| Control | 0 | 58304.5 | 38991 |
| Example 20 (0.32 µg/mℓ) | 0 | 31250 | 31250 |
| Example 21 (0.16 µg/mℓ) | 0 | 31250 | 31250 |
| Example 22 (1.60 µg/mℓ) | 0 | 17405 | 20897 |
| Example 25 (4.00 µg/mℓ) | 0 | 25557 | 16416 |
| Example 66 (200 µg/mℓ) | 0 | 25557 | 20897 |

**Table 5**

| | Day 0 (pg/mℓ) | Day 2 (pg/mℓ) | Day 4 (pg/mℓ) |
|---|---|---|---|
| Control | 0 | 435119 | 349386 |
| Example 28 (1.60 µg/mℓ) | 0 | 156250 | 69877 |
| Example 36 (3.20 µg/mℓ) | 0 | 104484 | 82082 |
| Example 46 (8.00 µg/mℓ) | 0 | 233662 | 156250 |
| Example 48 (1.60 µg/mℓ) | 0 | 82082 | 297434 |
| Example 53 (40.0 µg/mℓ) | 0 | 94822 | 233662 |

As is evident from Tables 1-5, the compounds according to the present invention have inhibitory activity against the production of p24 protein in HIV-infected cells and are useful as AIDS therapeutics.

### Industrial Applicability

The compounds according to the present invention have inhibitory activity against the production of p24 protein which makes up the shell of protein that protects the RNA of a virus, inhibit proliferation of a virus such as HIV in virus-infected cells, and are useful as pharmaceuticals such as virus proliferation inhibitors and AIDS therapeutics.

## Claims

1. An isoquinolinesulfonamide derivative represented by the following formula (1): 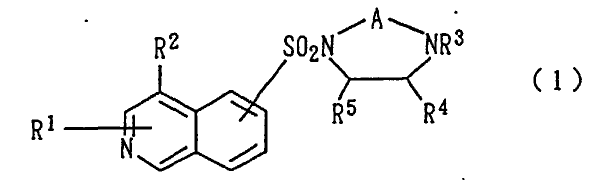 wherein A represents a linear or branched alkylene group, R¹ represents a hydrogen atom, a halogen atom, a hydroxyl group, an alkyl group, an alkoxy group, an aryloxy group, an benzyloxy which may be substituted by one or more halogen atoms, an alkylthio group, an amino group, an alkylamino group, a dialkylamino group or -O(CH₂)ₙNH₂ in which n stands for an integer of from 2 to 6, R² represents a hydrogen atom, a halogen atom, an alkyl group or a cyano group, R³ represents a hydrogen atom, an alkyl group, an acetyl group or a benzyloxycarbonyl group, and R⁴ and R⁵ may be the same or different and individually represent hydrogen atoms or lower alkyl groups; an N-oxide or salt thereof; or a solvate thereof.

2. A pharmaceutical comprising as an active ingredient an isoquinolinesulfonamide derivative, an N-oxide or salt thereof, or a solvate thereof according to claim 1.

3. A pharmaceutical according to claim 2, which is an AIDS therapeutic.

4. A viral proliferation inhibitor comprising as an active ingredient an isoquinolinesulfonamide derivative, an N-oxide or salt thereof, or a solvate thereof according to claim 1.

5. A pharmaceutical composition comprising an isoquinolinesulfonamide derivative, an N-oxide or salt thereof, or a solvate thereof according to claim 1 and a pharmacologically acceptable carrier.

6. Use of an isoquinolinesulfonamide derivative, an N-oxide or salt thereof, or a solvate thereof according to claim 1 as a pharmaceutical.

7. Use of an isoquinolinesulfonamide derivative, an N-oxide or salt thereof, or a solvate thereof according to claim 1 as an AIDS therapeutic.

8. Use of an isoquinolinesulfonamide derivative, an N-oxide or salt thereof, or a solvate thereof according to claim 1 as a viral proliferation inhibitor.

9. A treatment method for an HIV infectious disease, which comprises administering art effective amount of an isoquinolinesulfonamide derivative, an N-oxide or salt thereof, or a solvate thereof according to claim 1 to a patient.
